# EUROPÄISCHE PATENTSCHRIFT

(11) **EP 1 658 128 B1**
(45) Veröffentlichungstag und Bekanntmachung des Hinweises auf die Patenterteilung: **04.11.2009**
(21) Anmeldenummer: 04739595.9
(22) Anmeldetag: 04.06.2004
(51) Int. Cl.: B01F 13/00, B01F 5/04, B01J 19/00, A61K 8/00, A61K 9/00, B05B 11/00, B65D 83/14

(54) **MEHRKOMPONENTENVERPACKUNG MIT STATISCHEM MIKROMISCHER UND VERFAHREN ZUR HERSTELLUNG VON FORMULIERUNGEN**
MULTI-CONSTITUENT PACKAGING COMPRISING A STATIC MICROMIXER AND METHOD FOR PRODUCING FORMULATIONS
EMBALLAGE A PLUSIEURS CONSTITUANTS COMPORTANT UN MICROMELANGEUR STATIQUE ET PROCEDE POUR LA PRODUCTION DES FORMULATIONS

(30) Priorität: 25.07.2003 DE 10333924
(43) Veröffentlichungstag der Anmeldung: 24.05.2006
(73) Patentinhaber: The Procter and Gamble Company, Cincinnati, Ohio 45202 (US)
(72) Erfinder: SCHANZ, Gerhard, 64295 Darmstadt (DE); SENDELBACH, Gerhard, 64297 Darmstadt (DE)
(74) Vertreter: Hirsch, Uwe Thomas M.H.
(86) Internationale Anmeldenummer: PCT/EP2004/006041
(87) Internationale Veröffentlichungsnummer: WO 2005/018785

(56) Entgegenhaltungen:
- WO-A-00/54890
- WO-A-01/43857
- US-B- 6 494 614

## Beschreibung

Gegenstand der Erfindung ist ein Verpackungssystem mit mindestens zwei separaten Vorratskammern zur in situ Herstellung von Formulierungen aus mindestens zwei bis zur Anwendung voneinander getrennt gehaltenen Komponenten und einem integriertem statischen Mikromischer mit speziellen, plattenförmigen Mischerbauteilen.

Bei aus mehreren Stoffen bestehenden Anwendungsprodukten besteht häufig die Gefahr, dass die Produkte nicht über einen längeren Zeitraum stabil sind, weil einige der Inhaltsstoffe unerwünscht miteinander reagieren können. Aus diesem Grund enthalten die Produkte verschiedenste Additive. Die Additive haben die Nachteile, dass sie die Produkte verteuern, die Anwendungseigenschaften unerwünscht beeinflussen können, insbesondere Nebenwirkungen hervorrufen können. Um diese Probleme zu vermeiden, können die Produkte als Mehrkomponentenpräparate vorliegen, wobei nicht kompatible Inhaltstoffe in verschiedenen Komponenten enthalten sind, die erst unmittelbar vor der Anwendung vermischt werden. Mehrkomponentenpräparate werden auch bei anderen Anwendungen eingesetzt, wobei in einer ersten Formulierung geeignete Derivate oder Vorstufen der eigentlichen Wirkstoffe enthalten sind und die Wirkstoffe erst nach Vermischen mit einer zweiten Formulierung freigesetzt oder gebildet werden. Derartige Anwendungen sind z.B. die verzögerte Freisetzung oder Bildung pharmazeutischer oder kosmetischer Wirkstoffe, die Bildung von Oxidationshaarfarben aus Farbstoffvorstufen und Oxidationsmittel oder die verzögerte Aushärung von Klebstoffen oder Spachtelmassen nach Zugabe von geeigneten Härtern.

Die Anwendung von Mehrkomponentenpräparaten erfolgt häufig durch Entnahme aus getrennten Verpackungen oder getrennten Vorratskammern einer einzigen Verpackung und Vermischen durch Schütteln oder Rühren per Hand. Eine andere Möglichkeit besteht darin, die getrennten Formulierungen über ein geeignetes Fördersystem einer gemeinsamen Ausgabeöffnung zuzuführen, welche eine geeignete Vorrichtung zum Vermischen der Komponenten aufweist. Diese Systeme weisen häufig den Nachteil auf, das die Qualität, Konsistenz oder Wirksamkeit der Mischung unbefriedigend ist. Bei viskosen Medien können Inhomogenitäten auftreten, bei flüssigen, nichtviskosen Medien ist insbesondere die Bildung feindisperser Mischungen wie Emulsionen oder Mikroemulsionen häufig nicht möglich.

In der WO 00/54890, der WO 00/54735 und im SÖFW-Journal, 128. Jahrgang 11-2002, Seite 55 wird der Einsatz von statischen Mikromischern zur in-situ-Vermischung kosmetischer oder pharmazeutischer Formulierungen unmittelbar vor der Anwendung beschrieben. Die einzusetzenden Mikromischersysteme werden beschrieben in WO 01/43857, DE 195 11 603 (WO 96/30113, DE 197 46 583 (WO 99/20379), DE 197 46 584 (WO 99/20382), DE 197 46 585 (WO 99/20906), US-B-6494614 und DE 198 54 096 (WO 00/31422). Das Mischverfahren beruht darauf, dass die Komponenten durch sich wiederholt kreuzende Kanäle geleitet werden und multiplen Scherbedingungen der kommunizierenden Kanäle im Mikromischer ausgesetzt werden. Hierbei ist die Viskositätsdifferenz der zu vermischenden Medien kritisch: je größer sie ist, umso schlechter ist der Emulgierprozess. Insbsondere ist es schwierig, gute Emulsionen bei Verwendung viskoser Öle zu erhalten. Die beschriebenen Mischsysteme weisen eine relativ lange Mischstrecke auf, in welcher in Ruhestellung die unvollständig oder teilweise vermischten Komponenten verbleiben, was bei Inkompatibilitäten der Komponenten nachteilig ist. Die relativ langen Mikrokanäle führen außerdem zu einem relativ hohen Druckabfall, was durch erhöhte Kräfte bei der Förderung der Komponenten durch das Mischsystem kompensiert werden muss. Der Gegenstand der WO00/54890 offenbart ein Verpackungssystem gemäß dem Oberbegriff des Anspruchs 1.

Es ist daher wünschenswert, weitere, insbesondere verbesserte Systeme zur Vermischung von zwei oder mehr Komponenten unmittelbar vor der Anwendung zur Verfügung zu stellen, die diesen Nachteil nicht aufweisen.

Gelöst wird die Aufgabe durch ein Verpackungssystem mit mindestens zwei separaten Vorratskammern zur in situ Herstellung von Formulierungen aus mindestens zwei bis zur Anwendung voneinander getrennt gehaltenen Komponenten. Das Verpackungssystem weist mindestens einen statischen Mikromischer auf, welcher mindestens ein Bauteil in Form einer Platte aufweist und wobei die Platte
- mindestens eine Eintrittsöffnung für den Eintritt mindestens eines Eduktstroms in einen in der Plattenebene liegenden Verbindungskanal und mindestens eine Austrittsöffnung für den Austritt des Eduktstroms in eine in der Plattenebene liegende Mischzone aufweist,
- wobei die Eintrittsöffnung mit den Austrittsöffnungen durch den in der Plattenebene liegenden Verbindungskanal kommunizierend verbunden ist und
- wobei der Verbindungskanal vor der Mündung in die Mischzone durch Mikrostruktureinheiten in zwei oder mehr Teilkanäle aufgespalten wird, wobei die Breiten der Teilkanäle im Millimeter- bis Submillimeterbereich liegen und kleiner sind als die Breite der Mischzone (5).

Nachfolgend wird unter dem Begriff "Fluid" ein gasförmiger oder flüssiger Stoff oder ein Gemisch solcher Stoffe verstanden, das einen oder mehrere feste, flüssige oder gasförmige Stoffe gelöst oder dispergiert enthalten kann. Der Begriff Mischen umfasst auch die Vorgänge Lösen, Dispergieren und Emulgieren. Demzufolge umfasst der Begriff Mischung Lösungen, flüssig-flüssig-Emulsionen, gas-flüssig- und festflüssig-Dispersionen.

Der Begriff "Teilkanäle" umfaßt auch eine Aufspaltung des Eduktstroms in Teilströme durch Mikrostruktureinbauten unmittelbar vor dem Austritt in die Mischzone. Die Dimensionen, insbesondere die Längen und Breiten dieser Einbauten können dabei im Bereich von Millimetern liegen oder vorzugsweise kleiner 1 mm betragen. Die Teilkanäle sind vorzugsweise auf die zur Strömungskontrolle absolut nötige Länge verkürzt und erfordern daher für einen bestimmten Durchsatz vergleichbar geringe Drücke. Dabei kreuzen sich die Teilkanäle vorzugsweise nicht. Vorzugsweise liegt das Verhältnis der Länge zur Breite der Teilkanäle im Bereich von 1:1 bis 20:1, insbesondere von 8:1 bis 12:1, besonders bevorzugt etwa 10:1. Die Mikrostruktureinbauten sind vorzugsweise so ausgestaltet, dass die Strömungsgeschwindigkeit des Eduktstroms bei Austritt in die Mischzone sowohl größer ist als bei Eintritt in den Verbindungskanals und vorzugsweise auch größer ist als die Strömungsgeschwindigkeit des Produktstroms durch die Mischzone.

Die auf den Platten aufgebrachten Verbindungs- und Teilkanäle sind in Freiform ausführbar. Sowohl die Platten als auch jeder darauf enthaltene einzelne Kanal können in Höhe, Breite und Dicke variieren, um auch unterschiedliche Medien und Mengen fördern zu können. Die Grundform der Platten ist beliebig und kann rund z.B. kreisförmig oder elliptisch oder eckig, z.B. rechteckig oder quadratisch sein. Die Plattenform kann auch in Bezug auf eine möglichst einfache Herstellung oder in Bezug auf ein möglichst geringes Gewicht und eine möglichst geringe ungenutzte Fläche optimiert sein. Die Ausgänge der Teilkanäle können in jeder beliebigen Weise angeordnet sein, von der geraden Linie bis zur beliebigen geometrischen Form. Die Austrittsöffnungen können z.B. auf einer kreisförmigen Linie angeordnet sein, insbesondere wenn die Mischzone von der Plattenebene vollständig umschlossen vorliegt. Es lassen sich zwei bzw. mehr als zwei Komponenten (A, B, C usw.) in einer Scheibe führen und diese mit gleichen oder unterschiedlichen Mengenverhältnissen mischen. Die Teilkanäle können zueinander oder bezogen auf die Linie, auf der die Ausgänge in die Mischzone liegen, in beliebigen Winkeln verlaufen. Es können mehrere Teilkanäle nebeneinander angeordnet werden, die jeweils z.B. Komponente A führen und im benachbarten Abschnitt derselben Scheibe können mehrere Teilkanäle nebeneinander angeordnet werden, die jeweils z.B. Komponente B führen. Die Bauteile können mittels zusätzlicher Durchbrüche und zusätzlicher Teilkanäle in den Platten jedoch auch so gestaltet sein, dass sich von Teilkanal zu Teilkanal die Komponenten A, B usw. in derselben Platte abwechseln.

Die Teilkanäle weisen an der Mündung in die Mischzone bevorzugt eine Breite im Bereich von 1 µm bis 2 mm sowie eine Tiefe im Bereich von 10 µm bis 10 mm und besonders bevorzugt eine Breite im Bereich von 5 µm bis 250 µm sowie eine Tiefe im Bereich von 250 µm bis 5 mm auf.

Der Verbindungskanal kann eine variable Breite haben. Vorzugsweise ist das Verhältnis der größten Breite des Verbindungskanals und/oder der Breite der Eintrittsöffnung zur Breite der Teilkanäle an deren Austritt in die Mischzone größer 2, besonders bevorzugt größer 5. Das Verhältnis der Breite der Mischzone zur Breite der Teilkanäle ist vorzugsweise größer 2, besonders bevorzugt größer 5.

Die plattenförmigen Bauteile können eine Dicke von 10 bis 1000 µm haben. Die Höhe der Kanäle ist vorzugsweise kleiner 1000 µm, besonders bevorzugt kleiner 250 µm. Die Wanddicke der Mikrostruktureinbauten und des Kanalbodens ist vorzugsweise kleiner 100 µm, besonders bevorzugt kleiner 70 µm.

In einer besonderen Ausführungsform ist mindestens eine der Eintritts- oder Austrittsöffnungen oder die Mischzone von der Plattenebene vollständig umschlossen. Die Öffnungen liegen dann z.B. als runde oder eckige, z.B. rechteckige Ausnehmungen vor. Im Falle einer umschlossenen Mischzone ist die bevorzugte Form ellipsen- oder kreisförmig. Die Teilkanäle können sich in Form von Düsen in Richtung der Mischzone verjüngen. Die Teilkanäle können geradlinig oder spiralförmig gebogen sein. Die Teilkanäle können rechtwinklig in Bezug auf die Umfangslinie der Mischzone in die Mischzone münden oder in einem von 90° verschiedenen Winkel.

Bei nicht rechtwinkligem Verlauf sind bei der Bildung eines Stapels aus mehreren Mischerplatten vorzugsweise jeweils Platten mit entgegengesetzter Abweichung vom rechten Winkel benachbart. Ebenso sind bei spiralförmigem Verlauf der Teilkanäle bei der Bildung eines Stapels aus mehreren Mischerplatten vorzugsweise jeweils Platten mit entgegengesetzter Drehrichtung der Spirale benachbart.

Der Verbindungskanal zwischen den Öffnungen ist vorzugsweise durch eine Vertiefung ausgebildet. Die Eintritts- und/oder Austrittsöffnung oder die Mischzone können aber auch am Plattenrand oder durch Aussparungen am Plattenrand angeordnet sein.

In einer weiteren besonderen Ausführungsform sind mindestens zwei Eintrittsöffnungen für mindestens zwei verschiedene Edukte vorhanden, wobei jede Eintrittsöffnung durch je einen Verbindungskanal mit der Mischzone verbunden ist. Dabei liegen vorzugsweise zwei Austrittsöffnungen für zwei verschiedene Edukte an gegenüberliegenden Seiten der Mischzone, wobei die Mischzone vorzugsweise vollständig umschlossen innerhalb der Plattenebene positioniert ist.

Als Material für die Bauteile eignen sich z.B. Metalle, insbesondere korrosionsbeständige Metalle wie z.B. Edelstahl, sowie Gläser, Keramik oder Kunststoff. Die Bauteile können hergestellt werden durch an sich bekannte Techniken zur Erzeugung von Mikrostrukturen auf Oberflächen, z.B. durch Ätzen oder Fräsen von Metallen oder durch Prägen oder Spritzen von Kunststoffen.

Ein erfindungsgemäß einzusetzender statischer Mikromischer weist ein Gehäuse mit mindestens 2 Fluidzuführungen und mindestens einer Fluidabführung auf. In dem Gehäuse befinden sich ein oder mindestens 2 zu einem Stapel angeordnete plattenförmige Mikromischerbauteile. Aus einer beliebigen Anzahl an Platten können Stapel erzeugt werden, die einen der Stapelhöhe entsprechenden Durchfluß realisieren lassen. Um an jeder Stelle des Mischers denselben Druck zu gewährleisten, kann bei größeren Längen die Fluidzufuhr an mehreren Stellen erfolgen. Nuten oder Stege in bzw. auf den Platten können der Stapel- und Justierbarkeit dienen. Die Platten liegen so übereinander, dass die Eintrittsöffnungen Nebenkanäle zum Zuführen des jeweiligen Eduktstroms und die Austrittsöffnungen bzw. die Mischzonen zusammen einen Hauptkanal zum Abführen des Produktstroms bilden und sich die Haupt- und Nebenkanäle durch den Stapel erstrecken. Wenn die Eintrittsöffnungen als Aussparungen am Plattenrand angeordnet sind, kann die Gehäusewand einen den jeweiligen Nebenkanal nach außen abschließenden Teil der Nebenkanalwand bilden. Wenn die Mischzone als Aussparung am Plattenrand angeordnet ist, kann die Gehäusewand einen den Hauptkanal nach außen abschließenden Teil der Hauptkanalwand bilden. Insgesamt kann der Mikromischer z.B. mindestens 5, 10, 100 oder auch mehr als 1000 Teilkanäle aufweisen und besteht aus einem Stapel von mit jeweils mehreren Teilkanälen aufweisenden Platten.

Das Verpackungssystem kann eine geeignete Vorrichtung zur Förderung der getrennt gehaltenenen Komponenten durch den Mikromischer aufweisen. Hierbei kann es sich um eine Pumpvorrichtung handeln, die manuell oder elektrisch betrieben werden kann. Es sind aber auch durch Treibmittel oder Überdurck betriebene Fördervorrichtungen möglich.

Vorzugsweise ist jeder aus einer Austrittsöffnung einer Platte in die Mischzone austretende Teilstrom eines ersten Edukts A einem aus einer Austrittsöffnung einer benachbarten Platte in die Mischzone austretenden Teilstroms eines zweiten Edukts B unmittelbar benachbart und es kommt in der Mischzone zu einer Vermischung durch Diffusion und/oder Turbulenz.

In einer Ausführungsform des Mikromischers sind die Verbindungskanäle der Platten durch Vertiefungen ausgebildet und die Verbindungskanäle werden vor der Mündung in die Mischzone durch auf den Platten angebrachte Mikrostruktureinheiten in Teilkanäle aufgespalten. In einer alternativen Ausführungsform sind die Verbindungskanäle der Platten durch Ausnehmungen in den Platten gebildet, wobei die Platten als Zwischenplatten zwischen je einer Deck- und einer Bodenplatte angeordnet sind und die Verbindungskanäle vor der Mündung in die Mischzone durch an den Deck- und/oder Bodenplatten angebrachten Mikrostruktureinheiten in Teilkanäle aufgespalten werden.

Gegenstand der Erfindung ist auch ein in situ Verfahren zur Herstellung von aus mindestens zwei vorzugsweise fluiden Komponenten bestehenden Formulierungen unmittelbar vor der Anwendung. Mindestens zwei zunächst getrennt gehaltene, vorzugsweise fluide Eduktströme werden miteinander vermischt und die Vermischung erfolgt unter Verwendung mindestens eines der oben beschriebenen erfindungsgemäßen Bauteile, statischen Mikromischer bzw. Verpackungssysteme. Hierbei ist vorzugsweise die Strömungsgeschwindigkeit des Eduktstroms oder der Eduktströme in die Mischzone größer als die Strömungsgeschwindigkeit der Produktmischung innerhalb der Mischzone. Besonders bevorzugt sind Ausgestaltungen des Mischers sowie Strömungsgeschwindigkeiten, bei denen in der Mischzone Turbulenz erzeugt wird und die Mischung in der Mischzone zumindest teilweise durch Turbulenz erfolgt.

Das erfindungsgemäße Mischverfahren umfaßt insbesondere auch Verfahren zum Homogenisieren, zur Herstellung von Dispersionen, Emulsionen oder Lösungen sowie zum Begasen oder Aufschäumen von Flüssigkeiten. Hierfür wird eine kontinuierliche flüssige Phase mit mindestens einer unlöslichen, zu dispergierenden fluiden Phase oder mit mindestens einer löslichen fluiden Phase unter Verwendung mindestens eines erfindungsgemäßen Bauteils oder eines erfindungsgemäßen statischen Mikromischers vermischt. Die beiden Phasen können entweder über verschiedene Nebenkanäle zugeführt werden oder eine der Phasen (vorzugsweise die kontinuierliche Phase) wird durch den Hauptkanal und eine zweite Phase über einen Nebenkanal zugeführt.

In einer besonderen Ausführungsform handelt es sich um ein Mischverfahren für chemisch reaktive Inhaltsstoffe, wobei
- mindestens zwei zunächst getrennt gehaltene fluide Eduktströme, welche reaktionsfähige Komponenten enthalten oder daraus bestehen, miteinander vermischt werden und wobei
- während oder nach Vermischung spontan oder durch Energiezufuhr oder mittels geeigneter Katalysatoren induziert eine chemische Reaktion der Komponenten abläuft
und wobei die Vermischung unter Verwendung mindestens eines erfindungsgemäßen Bauteils oder mindestens eines erfindungsgemäßen statischen Mikromischers erfolgt.

Zur Erhöhung der Kapazität der erfindunsgemäßen Verfahren kann die Anzahl der Kanäle in den Platten erhöht werden oder die Anzahl der übereinandergeschichteten Platten in einem Mikromischer kann erhöht werden. Es können auch zwei oder mehrere Mikromischer in Reihe geschaltet hintereinander oder parallel geschaltet nebeneinander betrieben werden. Besonders vorteilhaft ist es, wenn dabei zunächst mit einem Mikromischer mit größeren Kanaldurchmessern eine gröbere Vormischung erzeugt wird und nachfolgende Mikromischer zunehmend kleinere Kanaldurchmesser aufweisen.

In einer besonderen Ausführungsform ist mindestens einer der Verpackungsteile für die einzelnen Komponenten separat austauschbar. Hierdurch ist es dem Anwender möglich, unterschiedliche Wirkstoffzusammensetzungen miteinander individuell zu kombinieren. Wenn eine erste Komponente unparfümiert ist, kann z.B. durch Austausch einer zweiten, parfümierten Komponente in einfacher Weise ein an die individuellen Bedürfnisse angepaßte, individuelle Produktparfümierung verwirklicht werden.

Nachfolgend werden beispielhafte Ausführungsformen erfindungsgemäßer Bauteile und Mikromischer anhand von Zeichnungen erläutert.
- Fig. 1a-b: Mischplatten mit zwei Eintrittsöffnungen für zwei Eduktströme wobei Ein- und Austrittsöffnungen umschlossen sind
- Fig. 1c: Mischplatte mit einer einzigen Eintrittsöffnung, wobei Ein- und Austrittsöffnungen umschlossen sind
- Fig. 1d: Mischplatte mit jeweils umschlossener Eintritts-, Durchtritts- und Austrittsöffnung
- Fig. 2a-c: Mischplatten mit drei Eintrittsöffnungen für bis zu drei verschiedene Eduktströme wobei Ein- und Austrittsöffnungen umschlossen sind
- Fig. 3a-b: Mischplatten mit zwei Eintrittsöffnungen am Plattenrand für zwei Eduktströme und umschlossener Austrittsöffnung
- Fig. 3c-d: Mischplatten mit vier Eintrittsöffnungen am Plattenrand für bis zu vier verschiedene Eduktströme und umschlossener Austrittsöffnung
- Fig. 4a-f: Mischplatten mit je einer umschlossenen Eintritts- und Durchtrittsöffnung für zwei Eduktströme und Austrittsöffnung am Plattenrand
- Fig. 5a-b: Mischplatten mit je einer umschlossenen Eintrittsöffnung und zwei umschlossenen Durchtrittsöffnungen für bis zu drei verschiedene Eduktströme und Austrittsöffnung am Plattenrand
- Fig. 6a: Längsschnitt des schematischen Aufbaus eines statischen Mikromischers
- Fig. 6b: Mischerscheibe in einem offenen Gehäuse
- Fig. 7a-b: Mischplatten mit umschlossenen Ein- und Durchtrittsöffnungen und zusätzlichen Teilkanälen, wobei benachbarte Teilkanäle von verschiedenen Edukten durchströmt werden können
- Fig. 8a,c: Mischplatten mit umschlossenen Ein- und Durchtrittsöffnungen und zusätzlichen Teilkanälen, wobei benachbarte Teilkanäle von verschiedenen Edukten durchströmt werden können
- Fig. 8b: Mischplatte mit umschlossener Eintrittsöffnung und drei umschlossenen Durchtrittsöffnungen und zusätzlichen Teilkanälen, wobei benachbarte Teilkanäle von verschiedenen Edukten durchströmt werden können
- Fig. 9: Mikromischer mit Gehäuse und einem Stapel aus mehreren Mischplatten
- Fig. 10: Querschnitte durch Stapel von Mischerplatten mit die Mischzone verschließbarem Formkörper

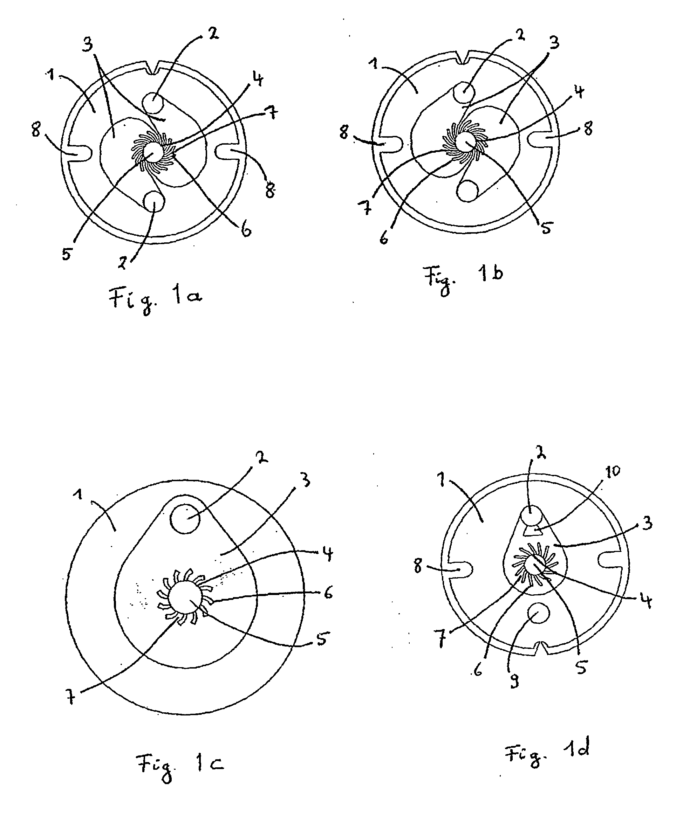

Eine Ausführungsform ist in Fig. 1a und Fig. 1b dargestellt. Die Platten (1) weisen je zwei umschlossene Eintrittsöffnungen (2) auf. Jede Eintrittsöffnung (2) ist mit je einem, in der Plattenebene durch eine Vertiefung ausgebildeten Verbindungskanal (3) verbunden. Jeder Vertiefungskanal (3) wird durch eine Vielzahl von Mikrostruktureinheiten (6) in eine Vielzahl von Teilkanälen (7) aufgespalten. Die Teilkanäle (7) münden durch die Austrittsöffnungen (4) in eine umschlossene Mischzone (5). Die Austrittsöffnungen (4) sind auf einer kreisförmigen Linie um die Mischzone (5) herum angeordnet. Mischzone (5) und Eintrittsöffnungen (2) sind als Durchbrüche in den Platten ausgebildet. Die Mikrostruktureinheiten sind beispielhaft spiralförmig gebogen ausgebildet, wobei die Spiralen in Fig.1a und Fig. 1b entgegengesetzten Drehsinn haben. Die Mikrostruktureinheitenkönnen aber auch geradlinig, ungebogen ausgebildet sein. Wenn die Platten rund ausgebildet sind, weisen sie vorzugsweise am Rand Aussparungen (8) auf, welche mit Halterungselementen (14) in einem Gehäuse (11) zusammenwirken können, um ein Verdrehen oder Verrutschen der Platten zu vermeiden. Die Platten können aber auch eckig, vorzugsweise viereckig, z.B. quadratisch ausgebildet sein. Dann können die Aussparungen und Halterungselemente entfallen. Durch die zwei Eintrittsöffnungen (2) können zwei verschiedene Eduktströme in einer Ebene der Mischzone (5) zugeführt werden, wobei die den beiden verschiedenen Eduktströmen zugeordneten Austrittsöffnungen vorzugsweise einander gegenüber liegen. Ein Mikromischer weist vorzugsweise einen Stapel von mehreren, aufeinander liegenden Bauteilen auf, wobei sich Platten gemäß Fig. 1a mit solchen gemäß Fig. 1b abwechseln und sich ein Aufbau mit alternierender Schichtstruktur ABAB usw. ergibt. Hierdurch wird erreicht, dass zwei verschiedene Eduktströme unmittelbar benachbart über- und untereinander der Mischzone (5) zugeführt werden können. In dem Stapel liegen die Platten so übereinander, dass die Eintrittsöffnungen Nebenkanäle zum Zuführen des jeweiligen Eduktstroms und die Mischzonen einen Hauptkanal zum Abführen des Produktstroms bilden. Über den Hauptkanal kann aber auch ein die spätere kontinuierliche Phase der Mischung bildendes Fluid zugeführt werden.

Eine weitere Ausführungsform ist in Fig. 1c dargestellt. Die Platte (1) weist eine einzige umschlossene Eintrittsöffnung (2) auf, welche mit einem in der Plattenebene durch eine Vertiefung ausgebildeten Verbindungskanal (3) verbunden ist. Der Vertiefungskanal (3) wird durch eine Vielzahl von Mikrostruktureinheiten (6) in eine Vielzahl von Teilkanälen (7) aufgespalten. Die Teilkanäle (7) münden durch die Austrittsöffnungen (4) in die Mischzone (5). Die Austrittsöffnungen (4) sind auf einer kreisförmigen Linie um die Mischzone (5) herum angeordnet. Mischzone (5) und Eintrittsöffnung (2) sind als Durchbrüche in der Platte ausgebildet. Die Mikrostruktureinheiten sind beispielhaft spiralförmig gebogen ausgebildet. Die Mikrostruktureinheiten können aber auch geradlinig, ungebogen oder in beliebigen anderen geometrischen Formen ausgebildet sein. Ein Mikromischer weist vorzugsweise einen Stapel von mehreren, aufeinander liegenden Bauteilen auf. In dem Stapel liegen die Platten so übereinander, dass die Eintrittsöffnungen einen Nebenkanal zum Zuführen eines Eduktstroms und die Mischzonen einen Hauptkanal zum Abführen des Produktstroms bilden. Über den Hauptkanal kann eine der zu vermischenden Komponenten, vorzugsweise ein die spätere kontinuierliche Phase der Mischung bildendes Fluid zugeführt werden. Diese Ausführungsform ist z.B. besonders geeignet zum Begasen von Flüssigkeiten, zum Aufschäumen von Flüssigkeiten mit Gasen oder zur Herstellung von Dispersionen. Hierbei wird die zu begasende Flüssigkeit bzw. das Dispersionsmedium über den zentralen Hauptkanal zugeführt und das Gas bzw. der zu dispergierende Stoff wird über den Nebenkanal zugeführt. Vorteilhafterweise kann der Plattenstapel einen Aufbau mit alternierender Schichtstruktur haben, wobei abwechselnd Platten aufeinanderliegen, die spiralförmige Mikrostruktureinheiten (6) mit entgegengesetzter Drehrichtung aufweisen. Es kann aber auch nur ein einziger Plattentyp verwendet werden. Die Mikrostruktureinheiten sind dann vorzugsweise geradlinig ausgebildet und so geformt, dass die Teilkanäle Düsen bilden.

Eine weitere Ausführungsform ist in Fig. 1d dargestellt.

Die Platte (1) weist eine umschlossene Eintrittsöffnung (2), eine umschlossene Mischzone (5) und eine umschlossene Durchtrittsöffnung (9) auf. Die Eintrittsöffnung (2) ist mit einem in der Plattenebene durch eine Vertiefung ausgebildeten Verbindungskanal (3) verbunden, welcher durch eine Vielzahl von Mikrostruktureinheiten (6) in eine Vielzahl von Teilkanälen (7) aufgespalten wird. Die Teilkanäle (7) münden durch die Austrittsöffnungen (4) in die Mischzone (5). Die Austrittsöffnungen (4) sind auf einer kreisförmigen Linie um die Mischzone (5) herum angeordnet. Mischzone (5), Eintrittsöffnung (2) und Durchtrittsöffnung (9) sind als Durchbrüche in der Platte ausgebildet. Die Mikrostruktureinheiten sind beispielhaft spiralförmig gebogen ausgebildet. Die Mikrostruktureinheiten können aber auch geradlinig, ungebogen oder in beliebigen anderen geometrischen Formen ausgebildet sein. Mit zusätzlichen Einbauten (10) im Verbindungskanal können die Strömungsverhältnisse im Verbindungskanal (3) optimiert werden. Wenn die Platten rund ausgebildet sind, weisen sie vorzugsweise am Rand Aussparungen (8) auf, welche mit Halterungselementen (14) in einem Gehäuse (11) zusammenwirken können, um ein Verdrehen oder Verrutschen der Platten zu vermeiden. Ein Mikromischer weist vorzugsweise einen Stapel von mehreren, aufeinanderliegenden Bauteilen auf, wobei Platten gemäß Fig. 1d abwechselnd um 180° verdreht übereinander liegen. Hierdurch wird erreicht, dass zwei verschiedene Eduktströme unmittelbar benachbart über- und untereinander der Mischzone (5) zugeführt werden können. In dem Stapel liegen die Platten so übereinander, dass sich Eintrittsöffnungen (2) und Durchtrittsöffnungen (9) abwechseln und zwei Nebenkanäle zum Zuführen von zwei Eduktströmen bilden und die Mischzonen einen Hauptkanal zum Abführen des Produktstroms bilden. Über den Hauptkanal kann aber auch ein die spätere kontinuierliche Phase der Mischung bildendes Fluid zugeführt werden. Vorteilhafterweise kann der Plattenstapel einen Aufbau mit alternierender Schichtstruktur haben, wobei abwechselnd Platten aufeinanderliegen, die spiralförmige Mikrostruktureinheiten (6) mit entgegengesetzter Drehrichtung aufweisen. Es kann aber auch nur ein einziger Plattentyp verwendet werden. Die Mikrostruktureinheiten sind dann vorzugsweise geradlinig ausgebildet und so geformt, dass die Teilkanäle Düsen bilden.

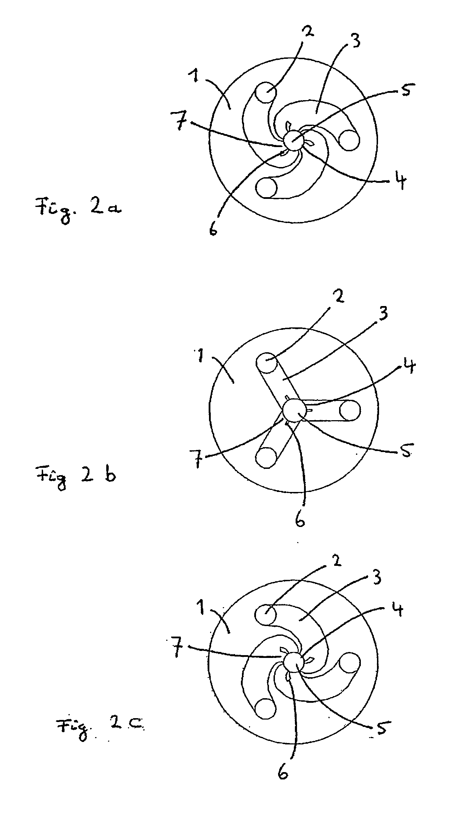

Eine weitere Ausführungsform ist in Fig. 2a bis 2c dargestellt. Die Platten (1) weisen je drei umschlossene Eintrittsöffnungen (2) auf. Jede Eintrittsöffnung (2) ist mit je einem, in der Plattenebene durch eine Vertiefung ausgebildeten Verbindungskanal (3) verbunden. Jeder Vertiefungskanal (3) wird durch mindestens eine Mikrostruktureinheit (6) in mindestens zwei Teilkanäle (7) aufgespalten. Durch eine größere Anzahl an Mikrostruktureinbauten kann eine Aufspaltung in eine entsprechend größere Anzahl an Teilkanälen erfolgen. Die Teilkanäle (7) münden durch die Austrittsöffnungen (4) in die Mischzone (5). Die Austrittsöffnungen (4) sind auf einer kreisförmigen Linie um die Mischzone (5) herum angeordnet. Mischzone (5) und Eintrittsöffnungen (2) sind als Durchbrüche in den Platten ausgebildet. Die Mikrostruktureinheiten können spiralförmig mit verschiedenen Drehrichtungen oder geradlinig ausgebildet sein. Durch die drei Eintrittsöffnungen (2) können gleiche oder bis zu drei verschiedene Eduktströme in einer Ebene der Mischzone (5) zugeführt werden. Ein Mikromischer weist vorzugsweise einen Stapel von mehreren, aufeinanderliegenden Bauteilen auf, wobei sich die verschiedenen Plattentypen gemäß Fig. 2a, 2b und 2c abwechseln und sich ein Aufbau mit alternierender Schichtstruktur, z.B. ABCABC ergibt. Hierdurch wird erreicht, dass jeweils zwei verschiedene Eduktströme unmittelbar benachbart über- und untereinander der Mischzone (5) zugeführt werden können. In dem Stapel liegen die Platten so übereinander, dass die Eintrittsöffnungen Nebenkanäle zum Zuführen des jeweiligen Eduktstroms und die Mischzonen einen Hauptkanal zum Abführen des Produktstroms bilden. Über den Hauptkanal kann aber auch ein die spätere kontinuierliche Phase der Mischung bildendes Fluid zugeführt werden.

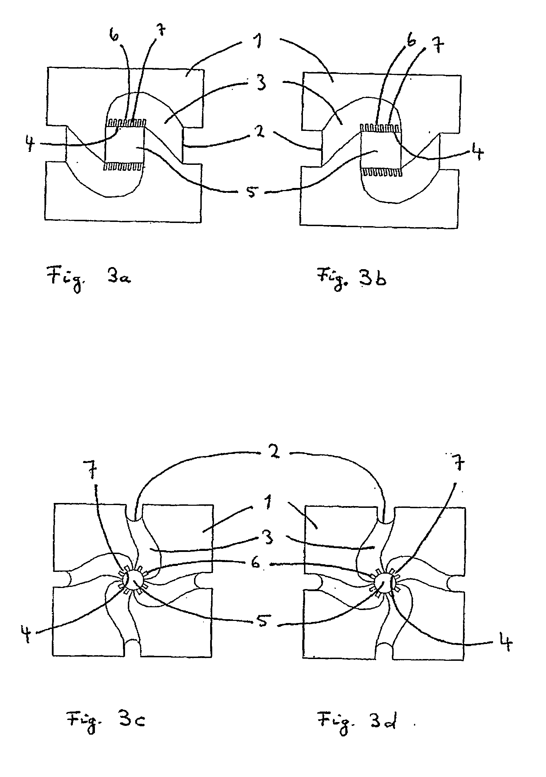

Eine weitere Ausführungsform ist in Fig. 3a und Fig. 3b dargestellt. Die Platten (1) weisen je zwei am Plattenrand positionierte Eintrittsöffnungen (2) auf. Jede Eintrittsöffnung (2) ist mit je einem, in der Plattenebene durch eine Vertiefung ausgebildeten Verbindungskanal (3) verbunden. Jeder Vertiefungskanal (3) wird durch eine Vielzahl von Mikrostruktureinheiten (6) in eine Vielzahl von Teilkanälen (7) aufgespalten. Die Teilkanäle (7) münden durch die Austrittsöffnungen (4) in eine umschlossene Mischzone (5). Die Austrittsöffnungen (4) sind auf einer geraden Linie angeordnet. Die Mischzone (5) ist beispielhaft als rechteckiger Durchbruch in den Platten ausgebildet. Die Mikrostruktureinheiten sind beispielhaft schräg zur Fließrichtung ausgebildet, wobei die Schrägen in Fig. 1a und Fig. 1b entgegengesetzte Richtung aufweisen. Die Mikrostruktureinheiten können aber auch jeweils mit gleicher oder keiner Schräge ausgebildet sein. Die Platten haben in etwa quadratische Grundform, können aber auch jede beliebige andere geometrische Grundform (eckig, rund, elliptisch etc.) haben. Durch die zwei Eintrittsöffnungen (2) können zwei verschiedene Eduktströme in einer Ebene der Mischzone (5) zugeführt werden, wobei die den beiden verschiedenen Eduktströmen zugeordneten Austrittsöffnungen bevorzugt einander gegenüber liegen. Ein Mikromischer weist vorzugsweise einen Stapel von mehreren, aufeinanderliegenden Bauteilen auf, wobei sich Platten gemäß Fig. 3a mit solchen gemäß Fig. 3b abwechseln und sich ein Aufbau mit alternierender Schichtstruktur ABAB ergibt. Hierdurch wird erreicht, dass zwei verschiedene Eduktströme unmittelbar benachbart über- und untereinander der Mischzone (5) zugeführt werden können. In dem Stapel liegen die Platten so übereinander, dass die Eintrittsöffnungen zusammen mit dem Mischergehäuse am Rand des Mischers Nebenkanäle zum Zuführen des jeweiligen Eduktstroms und die Mischzonen einen Hauptkanal im Innern des Mischers zum Abführen des Produktstroms bilden. Über den Hauptkanal kann aber auch ein die spätere kontinuierliche Phase der Mischung bildendes Fluid zugeführt werden.

Eine weitere Ausführungsform ist in Fig. 3c und Fig. 3d dargestellt. Die Platten (1) weisen je vier am Plattenrand positionierte Eintrittsöffnungen (2) auf. Jede Eintrittsöffnung (2) ist mit je einem, in der Plattenebene durch eine Vertiefung ausgebildeten Verbindungskanal (3) verbunden. Jeder Vertiefungskanal (3) wird durch mehrere Mikrostruktureinheiten (6) in mehrere Teilkanäle (7) aufgespalten. Die Teilkanäle (7) münden durch die Austrittsöffnungen (4) in eine umschlossene Mischzone (5). Die Austrittsöffnungen (4) sind auf einer Kreislinie angeordnet. Die Verbindungskanäle sind spiralförmig gebogen, wobei der Drehsinn der Spiralen in Fig. 3c und Fig. 3d entgegengesetzt sind. Die Mischzone (5) ist als Durchbruch in den Platten ausgebildet. Die Mikrostruktureinheiten sind beispielhaft gerade ausgebildet, können aber auch schräg oder spiralförmig gebogen sein. Die Platten haben in etwa quadratische Grundform, können aber auch jede beliebige andere geometrische Grundform (eckig, rund, elliptisch etc.) haben. Durch die vier Eintrittsöffnungen (2) können gleiche oder bis zu vier verschiedene Eduktströme in einer Ebene der Mischzone (5) zugeführt werden, wobei die verschiedenen Eduktströmen zugeordneten Austrittsöffnungen bevorzugt einander gegenüber liegen. Ein Mikromischer weist vorzugsweise einen Stapel von mehreren, aufeinanderliegenden Bauteilen auf, wobei sich Platten gemäß Fig. 3c mit solchen gemäß Fig. 3d mit entgegengesetztem Drehsinn der spiralartig gebogenen Verbindungskanäle abwechseln und sich ein Aufbau mit alternierender Schichtstruktur ABAB ergibt. Hierdurch wird erreicht, dass zwei verschiedene Eduktströme unmittelbar benachbart über- und untereinander der Mischzone (5) zugeführt werden können. In dem Stapel liegen die Platten so übereinander, dass die Eintrittsöffnungen zusammen mit dem Mischergehäuse am Rand des Mischers Nebenkanäle zum Zuführen des jeweiligen Eduktstroms und die Mischzonen einen Hauptkanal im Innern des Mischers zum Abführen des Produktstroms bilden. Über den Hauptkanal kann aber auch ein die spätere kontinuierliche Phase der Mischung bildendes Fluid zugeführt werden.

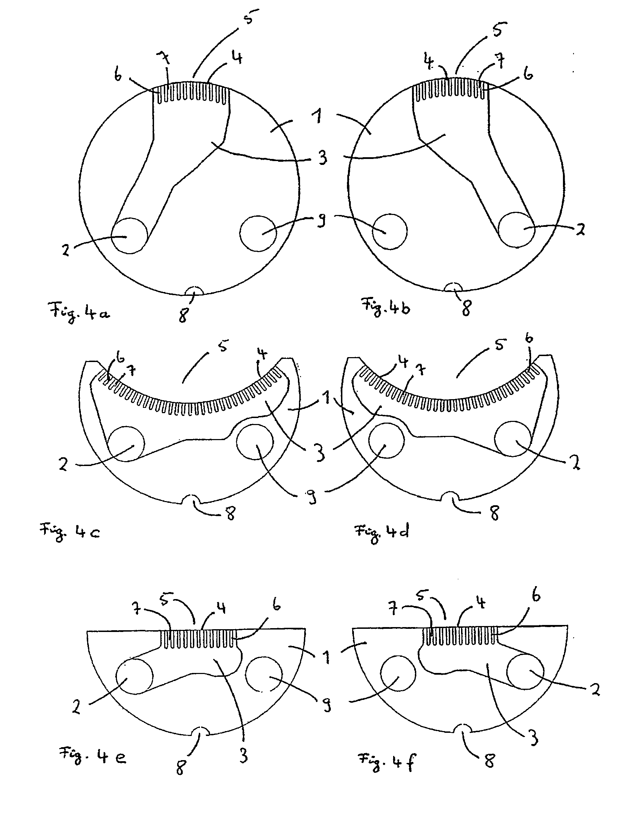

Weitere Ausführungsformen sind in Fig. 4a bis 4f dargestellt. Die Platten (1) weisen je eine umschlossene Eintrittsöffnung (2) und je eine umschlossene Durchtrittsöffnung (9) auf. Jede Eintrittsöffnung (2) ist mit je einem, in der Plattenebene durch eine Vertiefung ausgebildeten Verbindungskanal (3) verbunden. Jeder Verbindungskanal (3) wird durch eine Vielzahl von Mikrostruktureinheiten (6) in eine Vielzahl von Teilkanälen (7) aufgespalten. Die Teilkanäle (7) münden durch am Rand der Platten angeordnete Austrittsöffnungen (4) in eine außerhalb der Plattenfläche liegende Mischzone (5). Die Austrittsöffnungen (4) können auf geraden Linien (Fig. 4e, 4f) oder auf Bogensegmenten angeordnet sein, wobei die Bogensegmente konvex (Fig. 4a, 4b) oder konkav (Fig. 4c, 4d) sein können. Die Eintrittsöffnungen (2) und die Durchtrittsöffnungen (9) sind als Durchbrüche in den Platten ausgebildet. Die Mikrostruktureinheiten können parallel oder in verschiedenen Winkeln zur durch den Verbindungskanal vorgegebenen Fließrichtung angestellt sein. Wenn die Platten rund ausgebildet sind, weisen sie vorzugsweise am Rand Aussparungen (8) auf, welche mit Halterungselementen (14) in einem Gehäuse (11) zusammenwirken können, um ein Verdrehen oder Verrutschen der Platten zu vermeiden. Ein Mikromischer weist vorzugsweise einen Stapel von mehreren, aufeinanderliegenden Bauteilen auf, wobei sich Platten gemäß Fig. 4a mit solchen gemäß Fig. 4b, bzw. Platten gemäß Fig. 4c mit solchen gemäß Fig. 4d, bzw. Platten gemäß Fig. 4e mit solchen gemäß Fig. 4f jeweils abwechseln und sich ein Aufbau mit alternierender Schichtstruktur ABAB ergibt. Hierdurch wird erreicht, dass zwei verschiedene Eduktströme unmittelbar benachbart über- und untereinander der Mischzone (5) zugeführt werden können. Vorzugsweise sind die Winkel der Teilkanäle bei der Mündung in die Mischzone in Relation zur Umfangslinie der Mischzone in benachbarten Platten verschieden, besonders bevorzugt haben sie entgegengesetzte Abweichungen von 90°. In dem Stapel liegen die Platten so übereinander, dass sich Eintrittsöffnungen (2) und Durchtrittsöffnungen (9) abwechseln und zwei im Innern des Mischers liegende Nebenkanäle zum Zuführen von zwei Eduktströmen bilden. Die Mischzone kann mit einem Gehäuse einen Hauptkanal zum Abführen des Produktstroms bilden, sie kann aber auch zur Umgebung offen sein. Die nach außen offene Bauweise ist insbesondere bevorzugt, wenn die Mischung als Spray oder Schaum abgegeben werden soll, insbesondere, wenn sie mittels eines Gases versprüht oder aufgeschäumt wird.

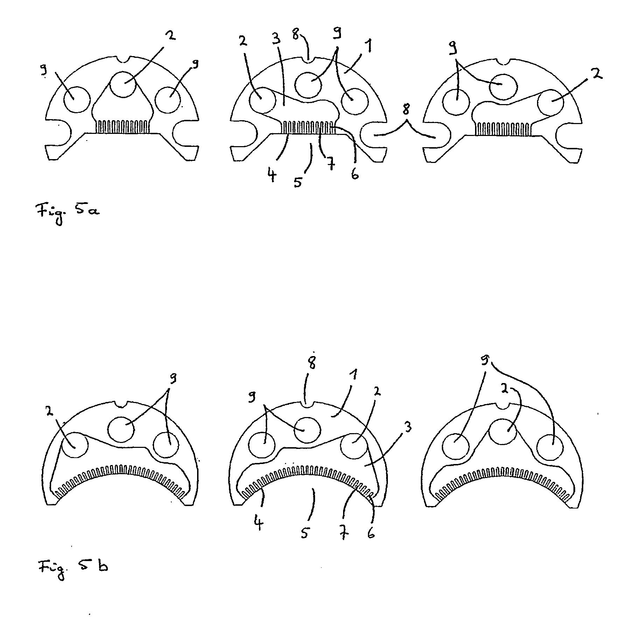

Weitere Ausführungsformen sind in Fig. 5a und Fig. 5b dargestellt. Die Platten (1) weisen je eine umschlossene Eintrittsöffnung (2) und je zwei umschlossene Durchtrittsöffnungen (9) auf. Jede Eintrittsöffnung (2) ist mit je einem, in der Plattenebene durch eine Vertiefung ausgebildeten Verbindungskanal (3) verbunden. Jeder Verbindungskanal (3) wird durch eine Vielzahl von Mikrostruktureinheiten (6) in eine Vielzahl von Teilkanälen (7) aufgespalten. Die Teilkanäle (7) münden durch am Rand der Platten angeordnete Austrittsöffnungen (4) in eine außerhalb der Plattenfläche liegende Mischzone (5). Die Austrittsöffnungen (4) können auf geraden Linien (Fig. 5a) oder auf Bogensegmenten (Fig. 5b) angeordnet sein, wobei die Bogensegmente konvex oder konkav sein können. Die Eintrittsöffnungen (2) und die Durchtrittsöffnungen (9) sind als Durchbrüche in den Platten ausgebildet. Die Mikrostruktureinheiten können parallel oder in verschiedenen Winkeln zur durch den Verbindungskanal vorgegebenen Fließrichtung angestellt sein. Wenn die Platten rund ausgebildet sind, weisen sie vorzugsweise am Rand Aussparungen (8) auf, welche mit Halterungselementen (14) in einem Gehäuse (11) zusammenwirken können, um ein Verdrehen oder Verrutschen der Platten zu vermeiden. Ein Mikromischer weist vorzugsweise einen Stapel von mehreren, aufeinander liegenden Bauteilen auf, wobei sich Platten der drei verschiedenen Typen gemäß Fig. 5a bzw. 5b jeweils abwechseln und sich ein Aufbau mit alternierender Schichtstruktur ABCABC ergibt. Hierdurch wird erreicht, dass jeweils verschiedene Eduktströme unmittelbar benachbart über- und untereinander der Mischzone (5) zugeführt werden können. Vorzugsweise sind die Winkel der Teilkanäle bei der Mündung in die Mischzone in Relation zur Umfangslinie der Mischzone in benachbarten Platten verschieden, besonders bevorzugt haben sie entgegengesetzte Abweichungen von 90°. In dem Stapel liegen die Platten (1) so übereinander, dass sich Eintrittsöffnungen (2) und Durchtrittsöffnungen (9) abwechseln und drei im Innern des Mischers liegende Nebenkanäle zum Zuführen von bis zu drei verschiedenen Eduktströmen bilden. Die Mischzone (5) kann mit einem Gehäuse einen Hauptkanal zum Abführen des Produktstroms bilden, sie kann aber auch zur Umgebung offen sein. Die nach außen offene Bauweise ist insbesondere bevorzugt, wenn die Mischung als Spray oder Schaum abgegeben werden soll, insbesondere, wenn sie mittels eines Gases versprüht oder aufgeschäumt wird.

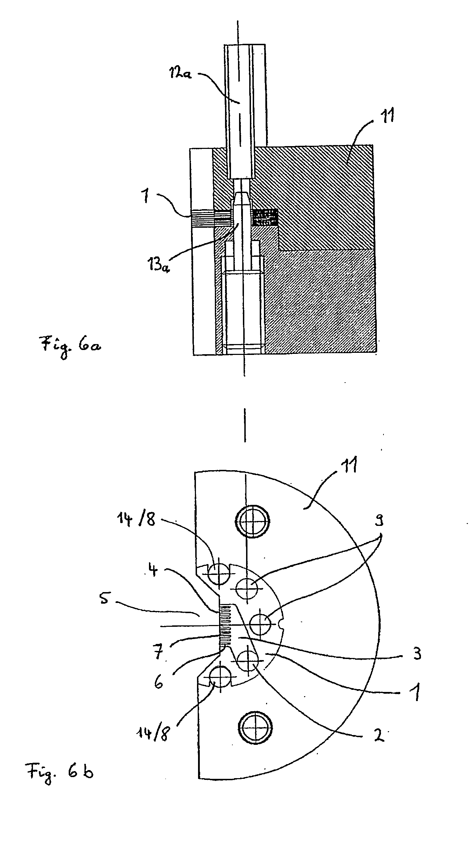

In Fig. 6a ist in Form eines Längsschnitts der schematische Aufbau einer Ausführungsform eines statischen Mikromischers dargestellt. Ein Gehäuse (11) weist Fluidzuführungen (12a) auf. In dem Gehäuse (11) ist ein Stapel aus mehreren erfindungsgemäßen Mischerplatten (1) enthalten. Die Ein- und/oder Durchtrittsöffnungen der Platten können mittels einer vorzugsweise senkrecht zur Plattenebene beweglichen Verschlußvorrichtung (13a) verschlossen und geöffnet werden. Mit der Verschlußvorrichtung kann auch die Strömungsgeschwindigkeit eingestellt werden. Die Mischung kann von einer innerhalb des Gehäuses liegenden Mischzone über eine geeignete Fluidabführung abgeführt werden oder sie kann bei einer außerhalb des Gehäuses liegenden Mischzone direkt abgegeben werden.

In Fig. 6b ist der Querschnitt eines statischen Mischers dargestellt. In einem Gehäuse (11) ist eine Mischerplatte (1) eingebaut, die mittels Aussparungen (8) und Halterungselementen (14) in Position gehalten wird. Als Mischerplatte ist beispielhaft eine solche gemäß Fig. 5a dargestellt.

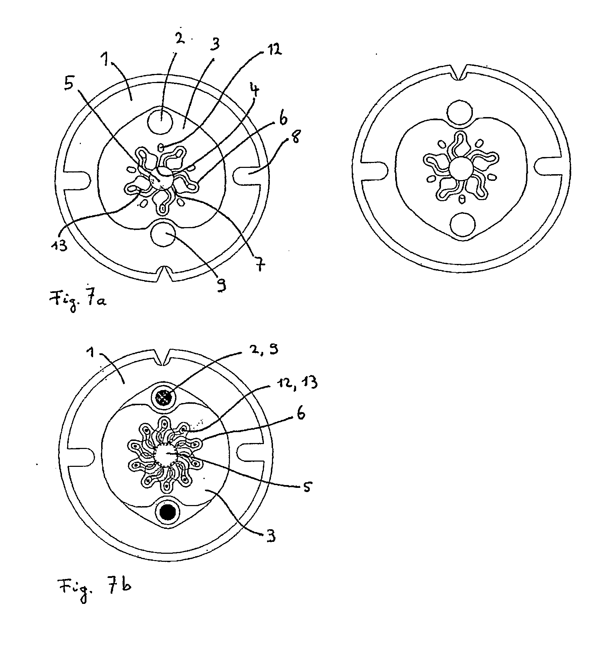

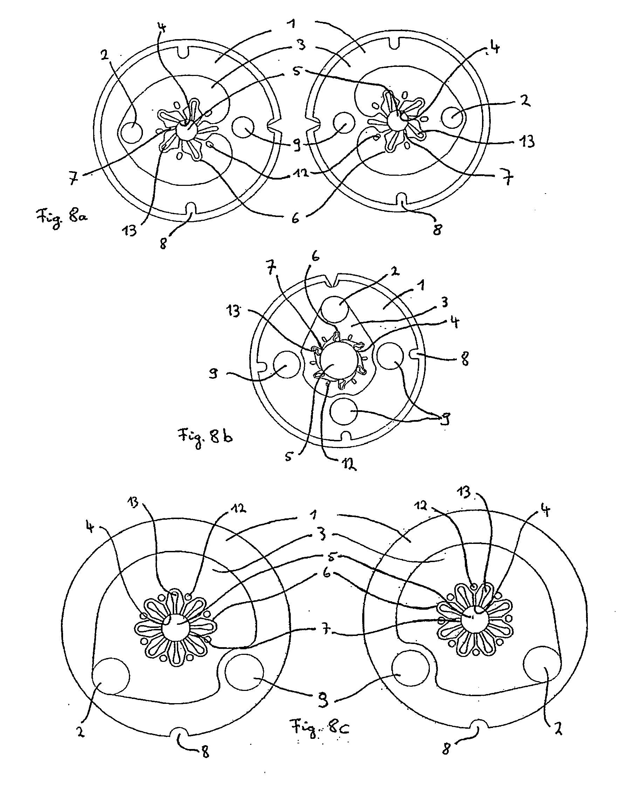

Weitere, bevorzugte Ausführungsformen sind in Fig. 7a-b und Fig. 8a-c dargestellt. Bei diesen Ausführungsformen weisen die Platten (1) nebeneinanderliegende Teilkanäle (7) und (13) auf, die abwechselnd von verschiedenen Eduktströmen durchströmt werden können und so verschiedene Eduktströme in einer Ebene unmittelbar benachbart der Mischzone (5) zugeführt werden können.

Die in Fig. 7a dargestellten Platten (1) weisen jeweils eine umschlossene Eintrittsöffnung (2), eine umschlossene Mischzone (5) und eine umschlossene Durchtrittsöffnung (9) auf. Die Eintrittsöffnung (2) ist mit einem in der Plattenebene durch eine Vertiefung ausgebildeten Verbindungskanal (3) verbunden, welcher durch eine Vielzahl von Mikrostruktureinheiten (6) in eine Vielzahl von Teilkanälen (7) aufgespalten wird. Die Teilkanäle (7) münden durch die Austrittsöffnungen (4) in die Mischzone (5). Die Austrittsöffnungen (4) sind auf einer kreisförmigen Linie um die Mischzone (5) herum angeordnet. Mischzone (5), Eintrittsöffnung (2) und Durchtrittsöffnung (9) sind als Durchbrüche in der Platte ausgebildet. In den Mikrostruktureinheiten (6) sind vertieft ausgebildete weitere Teilkanäle (13) integriert, welche gegenüber dem Verbindungskanal (3) abgeschirmt sind und in die Mischzone (5) münden. Die Teilkanäle (7) und die weiteren Teilkanäle (13) sind abwechselnd benachbart angeordnet. Die Platten weisen zusätzliche Durchbrüche (12) auf, wobei die Anzahl der Durchbrüche (12) und die Anzahl der zusätzlichen Teilkanäle (13) gleichgroß sind. Die Durchbrüche (12) sind so angeordnet, dass sie, wenn eine Platte (1) um 180° verdreht auf eine zweite Platte (1) gelegt wird, jeweils oberhalb der zusätzlichen Teilkanäle (13) der darunter liegenden Platte liegen. Ein durch die Eintrittsöffnung (2) in den Verbindungskanal (3) strömender Eduktstrom kann durch die Durchbrüche (12) in einen zusätzlichen Teilkanal (13) einer darunterliegenden Platte fließen. Die Winkel benachbarter Teilkanäle (7) und (13) zueinander und in Bezug auf die Umfangslinie der Mischzone können verschieden sein. In Fig. 7a haben die Winkel der Teilkanäle (7) gegenüber den Winkeln der zusätzlichen Teilkanäle (13) in Bezug auf die Umfangslinie der Mischzone (5) entgegengesetzte Abweichungen von 90°. Dadurch weisen die Austrittsöffnungen von je zwei Teilkanälen paarweise aufeinander zu. Dadurch können zwei verschiedene Eduktströme aufeinander zugeführt werden. Die Teilkanäle können aber auch parallel im rechten Winkel oder schräg zur Mischzone verlaufen. Fig. 7a zeigt nebeneinander zwei identische, um 180° verdrehte Platten (1). Fig 7b zeigt schematisch zwei um 180° verdreht aufeinandergelegte Platten. Ein Mikromischer weist vorzugsweise einen Stapel von mehreren, aufeinanderliegenden Bauteilen auf, wobei Platten gemäß Fig. 7a abwechselnd um 180° verdreht übereinander liegen. Hierdurch wird erreicht, dass zwei verschiedene Eduktströme sowohl unmittelbar benachbart über- und untereinander als auch unmittelbar benachbart nebeneinander der Mischzone (5) zugeführt werden können. In dem Stapel liegen die Platten so übereinander, dass sich Eintrittsöffnungen (2) und Durchtrittsöffnungen (9) abwechseln und zwei Nebenkanäle zum Zuführen von zwei Eduktströmen bilden und die Mischzonen einen Hauptkanal zum Abführen des Produktstroms bilden. Über den Hauptkanal kann aber auch ein die spätere kontinuierliche Phase der Mischung bildendes Fluid zugeführt werden. Außerdem liegen die Platten so übereinander, dass jeder zusätzliche Durchbruch (12) einer Platte mit je einem zugehörigen zusätzlichen Teilkanal (13) einer benachbarten Platte kommunizierend verbunden ist.

In Fig. 8a ist eine Ausführungsform ähnlich derjenigen der Fig. 7a dargestellt, mit dem Unterschied, dass die Teilkanäle (7) und die zusätzlichen Teilkanäle (13) parallel in gleichen Winkeln der Mischzone (5) schräg zugeführt werden. Die linke Platte der Fig. 8a unterscheidet sich dabei von der rechten Platte dadurch, dass der Winkel der Teilkanäle (7) und (13) zur Umfangslinie der Mischzone (5) eine entgegengesetzte Abweichung von 90° aufweist. Ein Mikromischer weist vorzugsweise einen Stapel von mehreren, aufeinander liegenden Bauteilen auf, wobei sich linke und rechte Platten gemäß Fig. 8a abwechseln und sich ein Aufbau mit alternierender Schichtstruktur ABAB ergibt. Hierdurch wird erreicht, dass zwei verschiedene Eduktströme unmittelbar benachbart über- und untereinander der Mischzone (5) in entgegensetzten Winkeln zugeführt werden können.

In Fig. 8c ist eine Ausführungsform ähnlich derjenigen der Fig. 8a dargestellt, mit dem Unterschied, dass die Teilkanäle (7) und die zusätzlichen Teilkanäle (13) parallel und senkrecht zur Mischzone (5) zugeführt werden. Ein Mikromischer weist vorzugsweise einen Stapel von mehreren, aufeinanderliegenden Bauteilen auf, wobei sich linke und rechte Platten gemäß Fig. 8c abwechseln und sich ein Aufbau mit alternierender Schichtstruktur ABAB ergibt. In dem Stapel liegen die Platten so übereinander, dass sich Eintrittsöffnungen (2) und Durchtrittsöffnungen (9) abwechseln und zwei Nebenkanäle zum Zuführen von zwei Eduktströmen bilden und die Mischzonen einen Hauptkanal zum Abführen des Produktstroms bilden. Außerdem liegen die Platten so übereinander, dass jeder zusätzliche Durchbruch (12) einer Platte mit je einem zugehörigen zusätzlichen Teilkanal (13) einer benachbarten Platte kommunizierend verbunden ist. Hierdurch wird erreicht, dass zwei verschiedene Eduktströme sowohl unmittelbar benachbart über- und untereinander als auch unmittelbar benachbart nebeneinander der Mischzone (5) zugeführt werden können.

Eine weitere Ausführungsform ist in Fig. 8b dargestellt. Eine Platte (1) weist eine umschlossene Eintrittsöffnung (2), drei umschlossene Durchtrittsöffnungen (9) und eine umschlossene Mischzone (5) auf. Die Eintrittsöffnung (2) ist mit einem in der Plattenebene durch eine Vertiefung ausgebildeten Verbindungskanal (3) verbunden, welcher durch eine Vielzahl von Mikrostruktureinheiten (6) in eine Vielzahl von Teilkanälen (7) aufgespalten wird. Die Teilkanäle (7) münden durch die Austrittsöffnungen (4) in die Mischzone (5). Die Austrittsöffnungen (4) sind auf einer kreisförmigen Linie um die Mischzone (5) herum angeordnet. Mischzone (5), Eintrittsöffnung (2) und Durchtrittsöffnung (9) sind als Durchbrüche in der Platte ausgebildet. In den Mikrostruktureinheiten (6) sind vertieft ausgebildete weitere Teilkanäle (13) integriert, welche gegenüber dem Verbindungskanal (3) abgeschirmt sind und in die Mischzone (5) münden. Die Teilkanäle (7) und die weiteren Teilkanäle (13) sind abwechselnd benachbart angeordnet. Die Platten weisen zusätzliche Durchbrüche (12) auf, wobei die Anzahl der Durchbrüche (12) und die Anzahl der zusätzlichen Teilkanäle (13) gleichgroß sind. Die Durchbrüche (12) sind so angeordnet, dass sie, wenn eine Platte (1) um 90° verdreht auf eine zweite Platte (1) gelegt wird, jeweils oberhalb der zusätzlichen Teilkanäle (13) der darunter liegenden Platte liegen. Ein durch die Eintrittsöffnung (2) in den Verbindungskanal (3) strömender Eduktstrom kann durch die Durchbrüche (12) in einen zusätzlichen Teilkanal (13) einer darunterliegenden Platte fließen. Die Winkel benachbarter Teilkanäle (7) und (13) zueinander und in Bezug auf die Umfangslinie der Mischzone können verschieden sein. In Fig. 8b haben die Winkel der Teilkanäle (7) gegenüber den Winkeln der zusätzlichen Teilkanäle (13) in Bezug auf die Umfangslinie der Mischzone (5) eine von 90° entgegengesetzte Abweichung. Dadurch weisen die Austrittsöffnungen von je zwei Teilkanäle paarweise aufeinander zu. Dadurch können zwei verschiedene Eduktströme aufeinander zugeführt werden. Die Teilkanäle können aber auch parallel im rechten Winkel oder schräg zur Mischzone verlaufen. Ein Mikromischer weist vorzugsweise einen Stapel von mehreren, aufeinanderliegenden Bauteilen auf, wobei Platten gemäß Fig. 8b in beliebiger Reihenfolge um 90°, 180° oder 270° verdreht übereinander liegen. Hierdurch wird erreicht, dass verschiedene Eduktströme sowohl unmittelbar benachbart über- und untereinander als auch unmittelbar benachbart nebeneinander der Mischzone (5) zugeführt werden können. Insgesamt können bis zu vier verschiedene Edukte mit dem Mikromischer vermischt werden. In dem Stapel liegen die Platten so übereinander, dass sich Eintrittsöffnungen (2) und Durchtrittsöffnungen (9) abwechseln und insgesamt vier Nebenkanäle zum Zuführen von bis zu vier Eduktströmen bilden und die Mischzonen einen Hauptkanal zum Abführen des Produktstroms bilden. Über den Hauptkanal kann aber auch ein die spätere kontinuierliche Phase der Mischung bildendes Fluid zugeführt werden. Außerdem liegen die Platten so übereinander, dass jeder zusätzliche Durchbruch (12) einer Platte mit je einem zugehörigen zusätzlichen Teilkanal (13) einer benachbarten Platte kommunizierend verbunden ist.

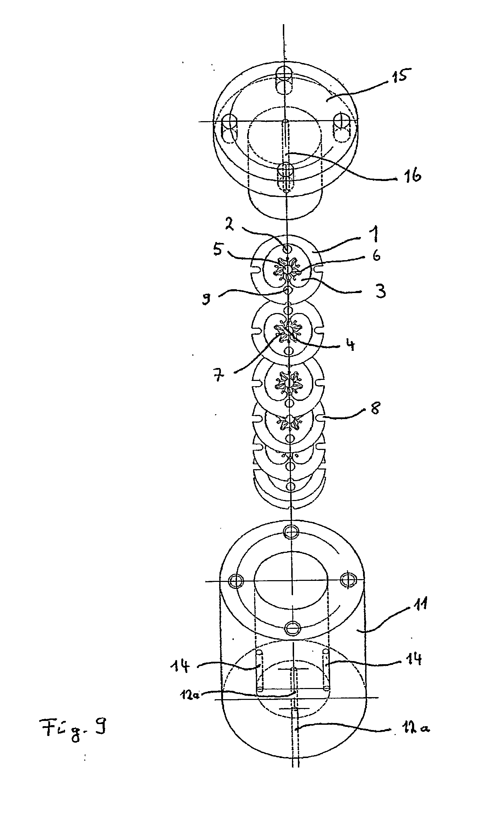

In Fig. 9 ist beispielhaft eine mögliche Ausführungsform eines erfindungsgemäß einsetzbaren Mikromischers in einer Explosionsdarstellung dargestellt. Ein Gehäuse (11) enthält einen Stapel an erfindungsgemäßen Bauteilen in Form von Platten (1). Dargestellt ist beispielhaft ein Stapel aus mehreren Platten gemäß Fig. 8a, es können aber auch andere erfindungsgemäße Platten verwendet werden, wobei gegebenenfalls die Gehäuseform, Anzahl und Position der Fluidzu- und abführungen etc. anzupassen sind. Die Platten (1) werden so eingesetzt, dass die Aussparungen (8) mit den Halterungselementen (14) zusammenwirken, um ein Verdrehen der Platten zu verhindern. Das Gehäuse weist zwei Fluidzuführungen (12a) zur Zuführung der Edukte auf. Das Gehäuse kann mit einem Deckel (15) verschlossen werden, welcher eine Fluidabführung (16) aufweist.

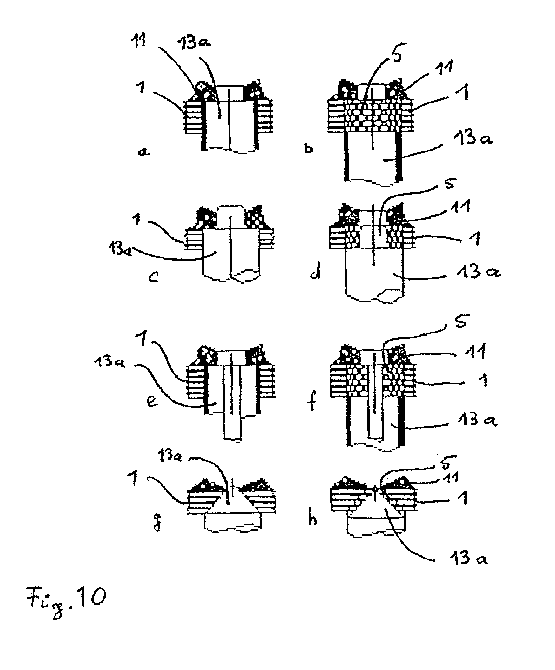

Fig. 10 zeigt weitere Ausführungsformen, bei denen die Mischzone (5) bzw. der durch die Mischzonen mehrerer plattenförmiger Bauteile (1) gebildete Mischraum in Ruhestellung durch eine die Austrittsöffnungen (4) verschließende Verschlußvorrichtung (13a) in Form eines Formkörpers ausgefüllt ist (Fig. 10 a, c, e, g). Durch einen geeigneten Mechanismus, z.B. bei Betätigung der Abgabevorrichtung des Verpackungssystems wird der Formkörper (13a) ganz oder teilweise aus der Mischzone (5) entfernt und die Austrittsöffnungen (4) werden ganz oder teilweise freigegeben (Fig. 10 b, d, f, h). Die Auslösung kann mittels vorwählbarem Druck und/oder mechanischer Zwangsführung erfolgen. Der Formkörper kann so gestaltet sein, dass er während des Dosier- und Mischvorgangs durch Druckaufbau und/oder geometrische Schikanen eine verstärkte Turbulenz mir verbesserter Mischungsqualität erzeugt. Nach erfolgter Dosierung kann der Formkörper wieder den Mischraum vollständig verschließen. Dadurch ist der Mischer frei von Mischungsrückständen, die ansonsten abreagieren und verderben könnten. Der Formkörper kann so in das Verpackungssystem integriert sein, dass er in Ruhestellung nach aussen bündig abschließt und eine glatte, gut sauber zu haltende Oberfläche entsteht (Fig. 10 a, b). Der Formkörper kann aber auch in Ruhestellung etwas nach aussen überstehen (Fig. 10 c). Er läßt sich dann im Falle einer Verklebung durch Eindrücken in das Gehäuse leicht lösen. Der Formkörper kann beliebige, an die Mischzonen (5) angepaßte Formen aufweisen, z.B. zylindrisch oder säulenförmig sein bei Mischzonen mit innerhalb eines Stapels gleichbleibender Öffnung (Fig. 10 a-f) oder konisch zulaufen (Fig. 10 g, h) bei Mischzonen, die innerhalb eines Stapels zur Produktabgabeöffnung sich verengende Öffnungen aufweisen.

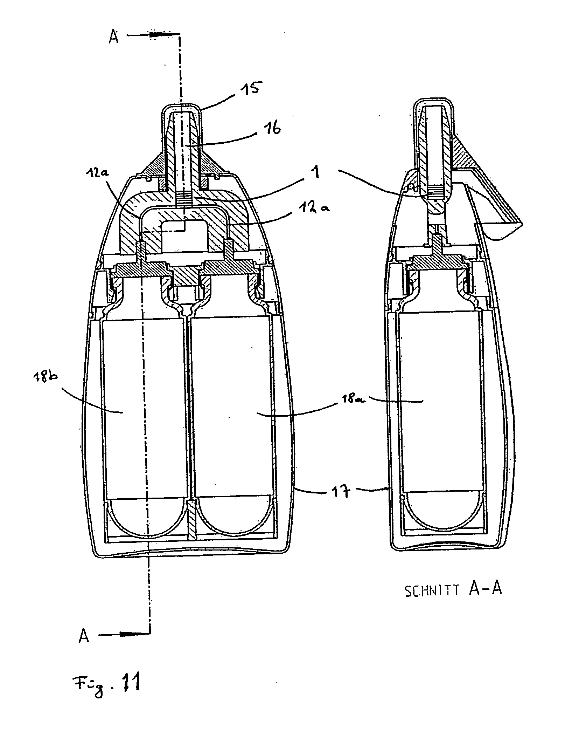

Fig. 11 zeigt einen Zweikomponentenbehälter mit integriertem Stapel von Mikromischerplatten. In einem mit einem Deckel 15 verscgließbaren äußeren Behälter 17 befinden sich zwei Innenbehälter 18a und 18 b, in denen zwei zu vermischende Zusammensetzungen bis zur Anwendung getrennt gehalten wrden können. Durch Betätigung eines geeigneten Abgabesystems werden die Zusammensetzungen über die Fluidzuführungen 12a einem Stapel von plattenförmigen Mischerbauteilen 1 zugeführt und vermischt. Die gebrauchsfertige Mischung tritt durch die Fluidabführung 16 aus.

Ein Vorteil des erfindungsgemäßen Verpackungssystems besteht darin, dass sich auch Komponenten mit unterschiedlichen Viskositäten gut vermischen lassen. Eine Ausführungsform betrifft daher ein Verpackungssystem, welches mindestens zwei getrennt gehaltene flüssige Komponenten mit unterschiedlichen Viskositäten enthält; d.h. das Verhältnis der Viskositäten derjenigen Komponente mit höherer Viskosität zu derjenigen mit niedrigerer Viskosität ist größer 1, vorzugsweise größer 1,5, insbesondere von 2 bis 100 (gemessen bei 25°C).

Das Verpackungsystem eignet sich vor allem zur Verwendung in Verfahren zum Mischen unmittelbar vor der Anwendung von Komponenten, die als fertige Mischung chemisch oder physikalisch instabil sind (Emulsionen, Dispersionen, Parfümierungen, verdickte Systeme wie Gele, Emulsionen mit pharmazeutischen Wirkstoffen, die in der fertigen Emulsion nicht lagerstabil sind, etc.). Für die kurze Zeit der Anwendung sind die hergestellten Mischungen für die jeweiligen Anwendungserfordernisse ausreichend stabil. Die Einzelkomponenten können durch geeignete Wahl des pH-Wertes oder anderer wirksamer Stabilisatoren stabilisiert sein, solange sie getrennt gehalten werden.

Anwendungsmöglichkeiten für kosmetische Präparate sind z.B.
- In situ-Erzeugung von Schampoos, Haarkuren, Haar- oder Hautmilch;
- Mischungen von Farbstoffvorstufen und Oxidationsmittel für Haarfarben; hier kann die fertige Mischung mit Hilfe eines Applikators direkt auf das Haar aufgetragen werden ohne die sonst übliche vorherige manuelle Vermischung in einer Schale;
- Mischungen von reaktiven Lösungen mit Verdickern, insbesondere viskosen Zubereitungen mit Gehalt an Oxidationsmitteln und Verdickern zur Blondierung von Haaren oder Fixierung von Dauerwellen;
- Erzeugung von Schäumen durch chemische Freisetzung von Gasen (z.B. CO₂ aus einer Carbonate oder Hydrogencarbonate enthaltenden Komponente und einer sauren Komponente);
- Schäume zur Haar- oder Hautbehandlung aus einer tensidhaltigen, flüssigen und einer gasförmigen Komponente;
- Vermischung von Endprodukten zur Erzielung besonderer Effekte, z.B. Farbwechselprodukten, bei denen nach Vermischung zeitverzögert eine chemische Farbwechselreaktion erfolgt, wobei die zeitliche Verzögerung auf die optimale Anwendungsdauer des Produktes, z.B. einer Haarkur abgestimmt ist;
- Erzeugung von Gelen aus dünnflüssigen Anfangskomponenten.
   Anwendungsmöglichkeiten für pharmazeutische Präparate sind z.B.
- wasserempfindliche Systeme erst im Moment der Anwendung aus einer wasserfreien und einer wasserhaltigen Komponente mischen;
- Salben, Emulsionen, Milchen etc. im Moment des Gebrauches frisch erzeugen, wobei die sonst üblichen, für eine längerfristige Emulsionsstabilität erforderlichen Emulgatoren reduziert oder eingespart werden können, wodurch die Verträglichkeit erhöht und Nebenwirkungen verringert werden können.

Anwendungsmöglichkeiten in der Klebetechnik sind z.B.
- Herstellung mehrkomponentiger Systeme im Moment des Gebrauchs, wobei die manuelle Vermischung einer ersten, aushärtbaren Komponente A und einer zweiten, einen Härter enthaltenden Komponente B entfällt; nach Abschluß des Mischvorgangs wird die Mischkammer vorzugsweise durch Verschließen mit einem Formkörper frei von aushärtenden Mischungsrückständen.

Anwendungsmöglichkeiten für Lebensmittel sind z.B.
- Erzeugung von Mayonnaisen, Senf, etc. im Moment des Gebrauchs;
- Homogenisierung von Milch, Milchprodukten etc.;
- Erzeugung von Sahne ohne mechanisches Schlagen.

Bei dem erfindungsgemäßem Verfahren ist in der Regel eine der zu vermischenden Phasen flüssig, die zweite und gegebenenfalls weitere Phasen können flüssig, fest oder gasförmig sein. Die zwei zu vermischenden Phasen werden in einem Mikromischer so zusammengeführt, dass die Komponenten an der Mischungszone am Austritt der Zuführungskanäle vermischt werden. Das erfindungsgemäße Verfahren eignet sich besonders zur unmittelbar vor der Anwendung erfolgenden Herstellung von Färbemitteln, Klebemitteln, Lebensmitteln, pharmazeutischen Mitteln, kosmetischen Mitteln oder Baustoffen, insbesondere zur Herstellung von emulsionsförmigen, mindestens einen haar- oder hautpflegenden kosmetischen, dermatologischen oder pharmazeutischen Wirkstoff enthaltenden Präparaten, haarfestigenden Mitteln, Haarfärbemitteln oder Dauerwellmitteln. Bei kosmetischen Anwendungen ist in mindestens einer Komponente mindestens ein haar- oder hautkosmetischer Inhaltsstoff enthalten. Bei diesem Inhaltsstoff kann es sich z.B. um einen haarpflegenden Stoff, einen haarfärbenden Stoff, einen haarfestigenden Stoff, einen Stoff mit Lichtschutzwirkung auf Haut und/oder Haar, einen Duftstoff, einen hautpflegenden Stoff, einen Antischuppenwirkstoff, einen haar- und/oder hautreinigenden Stoff oder um ein Konservierungsmittel handeln. Typische Wirkstoffmengen sind dabei 0,05 bis 20, vorzugsweise 0,14 bis 10 Gew.%.

Vorzugsweise ist eine der zu vermischenden Komponenten eine wässrige, flüssige Phase und die andere Komponente eine hydrophobe, flüssige oder eine einen wasserempfindlichen Stoff enthaltende Phase oder die Komponenten enthalten Stoffe, die bei Kontakt miteinander chemisch reagieren oder die physikalische Konsistenz der Mischung verändern.

Bei Dispersionen richtet sich der Anteil der einzuhomogenisierenden Phase an der fertigen Emulsion oder Suspension nach den Anforderungen des herzustellenden Endproduktes. Die lipophile Phase kann für Haarkuren beispielsweise von 2 bis 10 Gew.% oder für Cremes wie z.B. Haarfärbecremes auch bis ca. 50 Gew.% betragen. Die Homogenisierung kann emulgatorfrei erfolgen. Es kann aber auch ein Emulgator oder ein Tensid als Dispergierhilfe anwesend sein. Das Dispergierhilfsmittel kann in Mengen von 0,5 bis 30 Gew.% der fertigen Zusammensetzung vorliegen. Als Emulgatoren sind nicht-ionische, anionische, kationische, amphotere oder zwitterionische Emulgatoren geeignet. Geeignete Emulgatoren sind beispielsweise die im "International Cosmetic Ingredient Dictionary and Handbook", 7. Auflage, Band 2 im Abschnitt "Surfactants", insbesondere im Unterabschnitt "Surfactants - Emulsifying Agents" aufgeführten Emulgatoren. Nichtionische Emulgatoren sind z.B. oxethylierte Fettalkohole, oxethylierte Nonylphenole, Fettsäuremono- und -diglyceride, ethoxyliertes und hydriertes oder nicht hydriertes Rizinusöl, Fettsäurealkanolamide, oxethylierte Fettsäureester. Kationische Emulgatoren sind z.B. langkettige quaternäre Ammoniumverbindungen wie sie unter den CTFA-Bezeichnungen "Quaternium" bekannt sind wie z.B. Alkyltrimethylammoniumsalze oder Dialkyldimethylammoniumsalze mit C8- bis C22-Alkylgruppen. Anionische Emulgatoren sind z.B. Fettalkoholsulfate, Alkylethersulfate, Alkylbenzolsulfonate. Amphotere Emulgatoren sind z.B. Betaine wie Fettsäureamidoalkylbetaine und Sulfobetaine und C8- bis C22-Alkylbetaine.

Der Teilchendurchmesser der dispergierten Phase ist vorzugsweise kleiner 1 µm, besonders bevorzugt kleiner 0,2 µm. In einer weiteren Ausführungsform werden die Kanaldimensionen der Mikrobauteile eines Mikromischers sowie die Strömungs- und Druckverhältnisse so gewählt, dass bei der Emulgierung von wässriger und hydrophober Phase eine Mikro- oder Nanoemulsion entsteht, d.h. die Teilchengröße beträgt 100 nm oder weniger.

Die Dispergierung einer wässrigen Phase mit einer nicht mischbaren, hydrophoben Phase kann nach dem erfindungsgemäßen Verfahren mit oder ohne Emulgator erfolgen. Ein besonderer Vorteil des Verfahrens ist, dass kein oder wesentlich weniger Emulgator eingesetzt zu werden braucht, um eine Emulsion oder Dispersion einer bestimmten Viskosität zu erhalten, die nur kurzfristig, d.h. für die Zeit der Anwendung stabil zu sein braucht. Hierdurch wird das Reizpotential verringert und die Hautverträglichkeit verbessert. Wird ganz auf Emulgatoren verzichtet, so bilden sich metastabile Dispersionen mit gegenüber nach herkömmlichen Verfahren hergestellten Dispersionen verlängerter Haltbarkeit. Ein weiterer Gegenstand der Erfindung ist daher ein Verfahren zur Herstellung einer dispersionsförmigen Zubereitung wobei eine hydrophobe Phase in einem Mikromischer ohne Emulgator mit einer wässrigen Phase unmittelbar vor der Anwendung vermischt wird.

Ein weiterer Gegenstand der Erfindung ist ein Verfahren zur Herstellung von Reinigungsmitteln, insbesondere Haar-, Haut- oder Textilreinigungsmitteln, wobei die Zusammensetzungen mindestens ein waschaktives Tensid und gegebenenfalls weitere Zusätze enthalten. Bei den Haar- oder Hautreinigungsmittelzusammensetzungen handelt es sich um Shampoos, Duschbäder, Duschgele, Badepräparate etc. Eine bevorzugte Ausführungsform besteht darin, dass eine erste Komponente mindestens ein anionisches, waschaktives Tensid in einer wässrigen Phase enthält und eine zweite Komponente mindestens einen Pflegewirkstoff enthält, welcher bei längerer Lagerung nicht mit der ersten Komponente kompatibel ist, z.B. ein Öl oder einen kationischen Pflegestoff. Der Begriff 'wässrige Phase' umfaßt Wasser sowie Gemische von Wasser mit wasserlöslichen Lösungsmitteln wie niederen Alkoholen, z.B. Ethanol oder Isopropanol oder Polyolen wie Ethylenglykol, Diethylenglykol, Butylenglykol oder Glycerin, vorzugsweise jedoch Wasser. Bevorzugtes anionisches Tensid ist Alkylethersulfat. Geeignete Alkylethersulfate weisen eine Alkylgruppe mit 8 bis 22, bevorzugt von 10 bis 16 C-Atomen und einen Ethoxylierungsgrad von 1 bis 20, bevorzugt von 1 bis 4 auf. Besonders bevorzugt sind Laurylethersulfate. Geeignete Gegenionen sind Alkali- oder Erdalkaliionen z.B. Natrium-, Magnesium- oder auch Ammoniumionen. Geeignete Alkylethersulfate sind z.B. die im 'International Cosmetic Ingredient Dictionary and Handbook', 7. Auflage, Band 2 im Abschnitt 'Alkyl Ether Sulfates' aufgeführten Tenside.

Ein in der zweiten Komponente eines Reinigungsmittels einsetzbarer katonischer Pflegestoff ist eine Substanz, die auf Grund von kationischen oder kationisierbaren Gruppen, insbesondere protonierten Amingruppen oder quaternären Ammoniumgruppen eine Substantivität zu menschlichem Haar aufweist. Der kationische oder kationaktive haarpflegende Stoff ist vorzugsweise ausgewählt aus kationischen Polymeren, kationischen Tensiden, kationischen Silikonverbindungen, kationisch derivatisierten Proteinen, kationisch derivatisierten Proteinhydrolysaten und Betain mit jeweils mindestens einer kationischen oder kationaktiven Gruppe. Gute haarpflegende Wirkungen werden erzielt, wenn mindestens ein kationisches Polymer mit mindestens einem kationischen Tensid kombiniert wird. Zusätzlich kann noch mindestens eine kationische Silikonverbindung, insbesondere ein endständig diquaternäres Polydimethylsiloxan enthalten sein.

Geeignete kationische Tenside sind Tenside, welche eine quaternäre Ammoniumgruppe enthalten. Geeignete kationische Tenside sind insbesondere solche der allgemeinen Formel

N⁽⁺⁾R¹R²R³R⁴ X⁽⁻⁾

wobei R1 bis R4 unabhängig voneinander aliphatische Gruppen, aromatische Gruppen, Alkoxygruppen, Polyoxyalkylengruppen, Alkylamidogruppen, Hydroxyalkylgruppen, Arylgruppen oder Alkarylgruppen mit 1 bis 22 C-Atomen bedeuten, wobei mindestens einer der Reste R1 bis R4 mindestens 8 C-Atome aufweist und wobei X⁻ ein Anion darstellt, beispielsweise ein Halogen, Acetat, Phosphat, Nitrat oder Alkylsulfat, vorzugsweise ein Chlorid. Die aliphatischen Gruppen können zusätzlich zu den C-Atomen und den Wasserstoffatomen auch Querverbindungen oder andere Gruppen wie beispielsweise weitere Aminogruppen enthalten. Beispiele für geeignete kationische Tenside sind die Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, beispielsweise Lauryl- oder Cetylpyridiniumchlorid, Alkylamidoethyltrimethylammoniumethersulfate sowie Verbindungen mit kationischem Charakter wie Aminoxide, beispielsweise Alkylmethylaminoxide oder Alkylaminoethyldimethylaminoxide. Besonders bevorzugt ist Cetyltrimethylammoniumchlorid.

Bei den kationischen oder kationaktiven Polymeren handelt es sich um haarpflegende bzw. haarkonditionierende Polymere. Geeignete kationische Polymere enthalten vorzugsweise quaternäre Amingruppen. Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie z.B. Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen. Die Ammoniumgruppen enthaltenden Monomere können mit nicht kationischen Monomeren copolymerisiert sein. Geeignete Comonomere sind z.B. Acrylamid, Methacrylamid, Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Kationische Polymere mit quaternären Amingruppen sind z.B. die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes vinylpyrrolidon/Dimethylaminoethylmethacrylat Copolymer (Polyquaternium-11) sowie quaternäre Silikonpolymere bzw. -oligomere wie z.B. Silikonpolymere mit quaternären Endgruppen (Quaternium-80). Von den kationischen Polymeren ist z.B. Vinylpyrrolidon/Dimethylaminoethylmethacrylatmethosulfat Copolymer, das unter den Handelsbezeichnungen Gafquat^{®} 755 N und Gafquat^{®} 734 vertrieben wird und von denen das Gafquat^{®} 755 N besonders bevorzugt ist, geeignet. Weitere kationische Polymere sind beispielsweise das unter dem Handelsnamen LUVIQUAT^{®} HM 550 vertriebene Copolymer aus Polyvinylpyrrolidon und Imidazoliminmethochlorid, das unter dem Handelsnamen Merquat^{®} Plus 3300 vertriebene Terpolymer aus Dimethyldiallylammoniumchlorid, Natriumacrylat und Acrylamid, das unter dem Handelsnamen Gaffix^{®} VC 713 vertriebene Terpolymer aus Vinylpyrrolidon, Dimethylaminoethylmethacrylat und Vinylcaprolactam und das unter dem Handelsnamen Gafquat^{®} HS 100 vertriebene vinylpyrrolidon/Methacrylamidopropyltrimethylammoniumchlorid Copolymer.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel

G-O-B-N⁺R⁵R⁶R⁷ X⁻

G ist ein Anhydroglucoserest, beispielsweise Stärke- oder Celluloseanhydroglucose;
B ist eine divalente Verbindungsgruppe, beispielsweise Alkylen, Oxyalkylen, Polyoxyalkylen oder Hydroxyalkylen;
R⁵, R⁶ und R⁷ sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in R⁵, R⁶ und R⁷ vorzugsweise maximal 20 ist;
X ist ein übliches Gegenanion, hat die gleiche Bedeutung wie oben und ist vorzugsweise Chlorid. Eine kationische Cellulose wird unter der Bezeichnung Polymer JR von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Ein geeignetes kationisches Guarderivat wird unter der Handelsbezeichnung Jaguar^{®} R vertrieben und hat die INCI-Bezeichnung Guar Hydroxypropyltrimonium Chloride. Besonders bevorzugte kationaktive Stoffe sind Chitosan, Chitosansalze und Chitosan-Derivate. Bei den erfindungsgemäß einzusetzenden Chitosanen handelt es sich um vollständig oder partiell deacetylierte Chitine. Das Molekulargewicht des Chitosans kann über ein breites Spektrum verteilt sein, beispielsweise von 20.000 bis ca. 5 Millionen g/mol. Geeignet ist beispielsweise ein niedermolekulares Chitosan mit einem Molekulargewicht von 30.000 bis 70.000 g/mol. Vorzugsweise liegt das Molekulargewicht jedoch über 100.000 g/mol, besonders bevorzugt von 200.000 bis 700.000 g/mol. Der Deacetylierungsgrad beträgt vorzugsweise 10 bis 99%, besonders bevorzugt 60 bis 99%. Ein geeignetes Chitosan wird beispielsweise von der Firma Kyowa Oil&Fat, Japan, unter dem Handelsnamen Flonac^{®} vertrieben. Es hat ein Molekulargewicht von 300.000 bis 700.000 g/mol und ist zu 70 bis 80% entacetyliert. Ein bevorzugtes Chitosansalz ist Chitosoniumpyrrolidoncarboxylat, welches z.B. unter der Bezeichnung Kytamer^{®} PC von der Firma Amerchol, USA, vertrieben wird. Das enthaltene Chitosan hat ein Molekulargewicht von ca. 200.000 bis 300.000 g/mol und ist zu 70 bis 85% entacetyliert. Als Chitosanderivate kommen quaternisierte, alkylierte oder hydroxyalkylierte Derivate, beispielsweise Hydroxyethyl-, Hydroxypropyl- oder Hydroxybutylchitosan in Betracht. Die Chitosane oder Chitosanderivate liegen vorzugsweise in neutralisierter oder partiell neutralisierter Form vor. Der Neutralisationsgrad für das Chitosan oder das Chitosanderivat liegt vorzugsweise bei mindestens 50%, besonders bevorzugt zwischen 70 und 100%, bezogen auf die Anzahl der freien Basengruppen. Als Neutralisationsmittel können prinzipiell alle kosmetisch verträglichen anorganischen oder organischen Säuren verwendet werden wie beispielsweise Ameisensäure, Äpfelsäure, Milchsäure, Pyrrolidoncarbonsäure, Salzsäure u.a., von denen die Pyrrolidoncarbonsäure und die Milchsäure besonders bevorzugt sind.

Bevorzugt sind solche Polymere, die eine ausreichende Löslichkeit in Wasser oder in Wasser/Alkohol-Gemischen besitzen, um in der erfindungsgemäßen hydrophilen Phase in vollständig gelöster Form vorzuliegen. Die kationische Ladungsdichte beträgt vorzugsweise 0,2 bis 7 meq/g oder 0,4 bis 5 meq/g, insbesondere 0,6 bis 2 meq/g. In herkömmlichen Pflegeshampoos sind üblicherweise nur geringe Mengen an Kationpolymeren mit geringer kationischer Ladungsdichte (z.B. bis 3 meq/g) stabil einarbeitbar. Demgegenüber können erfindungsgemäß größere Mengen dieser gering kationisierten Polymere oder Polymere mit höherem Kationisierungsgrad (z.B. > 3 meq/g) eingesetzt werden.

Geeignete kationaktive Silikonvberbindungen weisen vorzugsweise entweder mindestens eine Aminogruppe oder mindestens eine Ammoniumgruppe auf. Geeignete Silikonpolymere mit Aminogruppen sind unter der INCI-Bezeichnung Amodimethicone bekannt. Hierbei handelt es sich um Polydimethylsiloxane mit Aminoalkylgruppen. Die Aminoalkylgruppen können seiten- oder endständig sein. Geeignete Aminosilikone sind solche der allgemeinen Formel

R⁸R⁹R¹⁰Si- (OSiR¹¹R¹²) - (OSiR¹³Q) -OSiR¹⁴R¹⁵R¹⁶

R⁸ , R⁹ , R¹⁴ und R¹⁵ sind unabhängig voneinander gleich oder verschieden und bedeuten C1- bis C10-Alkyl, Phenyl, Hydroxy, Wasserstoff, C1- bis C10-Alkoxy oder Acetoxy, vorzugsweise C1-C4-Alkyl, besonders bevorzugt Methyl;
R¹⁰ und R¹⁶ sind unabhängig voneinander gleich oder verschieden und bedeuten (CH₂)ₐ-NH₂ mit a gleich 1 bis 6, C1- bis C10-Alkyl, Phenyl, Hydroxy, Wasserstoff, C1- bis C10-Alkoxy oder Acetoxy, vorzugsweise C1-C4-Alkyl, besonders bevorzugt Methyl;
R¹¹ R¹² und R¹³ sind unabhängig voneinander gleich oder verschieden und bedeuten Wasserstoff, C1- bis C20-Kohlenwasserstoff, welcher O- und N-Atome enthalten kann, vorzugsweise C₁- bis C10-Alkyl oder Phenyl, besonders bevorzugt C1- bis C4-Alkyl, insbesondere Methyl;
Q bedeutet -A-NR¹⁷R¹⁸, oder -A-N⁺R¹⁷R¹⁸R¹⁹ wobei A für eine divalente C1- bis C20-Alkylenverbindungsgruppe steht, welche auch O- und N-Atome sowie OH-Gruppen enthalten kann, und
R¹⁷ , R¹⁸ und R¹⁹ unabhängig voneinander gleich oder verschieden sind und Wasserstoff, C1- bis C22-Kohlenwasserstoff, vorzugsweise C1- bis C-4-Alkyl oder Phenyl bedeuten. Bevorzugte Reste für Q sind - (CH₂)₃-NH₂, - (CH₂)₃NHCH₂CH₂NH₂, - (CH₂) ₃OCH₃CHOHCH₂NH₂ und - (CH₂)₃N (CH₂CH₂OH)₂, - (CH₂) ₃-NH₃⁺ und - (CH₂)₃OCH₂CHOHCH₂N⁺(CH₃)₂R²⁰, wobei R²⁰ ein C1- bis C22-Alkylrest ist, der auch OH-Gruppen aufweisen kann.
x bedeutet eine Zahl zwischen 1 und 10.000, vorzugsweise zwischen 1 und 1.000;
y bedeutet eine Zahl zwischen 1 und 500, vorzugsweise zwischen 1 und 50.

Das Molekulargewicht der Aminosilikone liegt vorzugsweise zwischen 500 und 100.000. Der Aminanteil (meq/g) liegt vorzugsweise im Bereich von 0,05 bis 2,3, besonders bevorzugt von 0,1 bis 0,5. Besonders bevorzugt sind silikonpolymere mit zwei endständigen quaternären Ammoniumgruppen. Diese Verbindungen sind unter der INCI-Bezeichnung Quaternium-80 bekannt. Hierbei handelt es sich um Polydimethylsiloxane mit zwei endständigen Alkylammoniumgruppen. Geeignete quaternäre Aminosilikone sind solche der allgemeinen Formel

R²¹R²²R²³N⁺-A-SiR⁸R⁹- (OSiR¹¹R¹²)ₙ-OSiR⁸R⁹-A-N⁺R²¹R²²R²³ 2X⁻

A hat die gleiche Bedeutung wie oben angegeben und ist vorzugsweise - (CH₂) ₃OCH₂CHOHCH₂N⁺ (CH3) ₂R²⁰, wobei R²⁰ ein C1- bis C22-Alkylrest ist, der auch OH-Gruppen aufweisen kann; R⁸, R⁹, R¹¹ und R¹² haben die gleiche Bedeutung wie oben angegeben und sind vorzugsweise Methyl;
R²¹, R²², und R²³ bedeuten unabhängig voneinander C1-bis C22-Alkylreste, welche Hydroxygruppen enthalten können und wobei vorzugsweise mindestens einer der Reste mindestens 10 C-Atome aufweist und die übrigen Reste 1 bis 4 C-Atome aufweisen; n ist eine Zahl von 0 bis 200, vorzugsweise von 10 bis 100. Derartige diquaternäre Polydimethylsiloxane werden von der Firma GOLDSCHMIDT/Deutschland unter den Handelsnamen Abil^{®} Quat 3270, 3272 und 3274 vertrieben.

Weitere geeignete kationaktive, haarpflegende Verbindungen sind kationisch modifizierte Proteinderivate oder kationisch modifizierte Proteinhydrolysate und sind z.B. bekannt unter den INCI-Bezeichnungen Lauryldimonium Hydroxypropyl Hydrolyzed Wheat Protein, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Silk, Lauryldimonium Hydroxypropyl Hydrolyzed Soy Protein oder Hydroxypropyltrimonium Hydrolyzed Wheat, Hydroxypropyltrimonium Hydrolyzed Casein, Hydroxypropyltrimonium Hydrolyzed Collagen, Hydroxypropyltrimonium Hydrolyzed Keratin, Hydroxypropyltrimonium Hydrolyzed Rice Bran Protein, Hydroxypropyltrimonium Hydrolyzed Silk, Hydroxypropyltrimonium Hydrolyzed Soy Protein, Hydroxypropyltrimonium Hydrolyzed Vegetable Protein. Geeignete kationisch derivatisierte Proteinhydrolysate sind Substanzmischungen, die beispielsweise durch Umsetzung von alkalisch, sauer oder enzymatisch hydrolysierten Proteinen mit Glycidyltrialkylammoniumsalzen oder 3-Halo-2-hydroxypropyltrialkylammoniumsalzen erhalten werden können. Proteine, die als Ausgangsstoffe für die Proteinhydrolysate dienen, können sowohl pflanzlicher als auch tierischer Herkunft sein. Übliche Ausgangsstoffe sind beispielsweise Keratin, Collagen, Elastin, Sojaprotein, Reisprotein, Milchprotein, Weizenprotein, Seidenprotein oder Mandelprotein. Durch die Hydrolyse entstehen Stoffmischungen mit Molmassen im Bereich von ca. 100 bis ca. 50.000. Übliche mittlere Molmassen liegen im Bereich von etwa 500 bis etwa 1000. Vorteilhafterweise enthalten die kationisch derivatisierten Proteinhydrolysate eine oder zwei lange C8- bis C22-Alkylketten und entsprechend zwei oder eine kurze C1- bis C4-Alkylketten. Verbindungen mit einer langen Alkylkette sind bevorzugt.

Ein in der zweiten Komponente eines Reinigungsmittels als Pflegewirkstoff einsetzbares Öl ist eine bei Raumtemperatur (25°C) flüssige, hydrophobe Substanz. Der Gehalt kann von 0,1 bis 20 Gew.%, besonders bevorzugt von 1 bis 10 Gew.% betragen. Die zweite Komponente kann als Voremulsion des Öls in Wasser vorliegen. Bei der hydrophoben Substanz kann es sich um einen leicht flüchtigen oder um einen schwerflüchtigen Stoff handeln. Die leicht flüchtigen hydrophoben Stoffe sind bei Raumtemperatur flüssig und weisen einen Siedepunkt im Bereich von vorzugsweise 30 bis 250°C, besonders bevorzugt von 60 bis 220 °C auf. Geeignet sind z.B. flüssige Kohlenwasserstoffe, flüssige cyclische oder lineare Silikone (Dimethylpolysiloxane) oder Gemische der genannten Stoffe. Geeignete Kohlenwasserstoffe sind Paraffine oder Isoparaffine mit 5 bis 14 C-Atomen, besonders bevorzugt mit 8 bis 12 C-Atomen, insbesondere Dodekan oder Isododekan. Geeignete flüssige, leicht-flüchtige Silikone sind cyclische Dimethylsiloxane mit 3 bis 8, vorzugsweise 4 bis 6 Si-Atomen, insbesondere Cyclotetradimethylsiloxan, cyclopentadimethylsiloxan oder Cyclohexadimethylsiloxan. Geeignet sind weiterhin Dimethylsiloxan/Methylalkylsiloxan Cyclocopolymere, z.B. Silicone FZ 3109 von Union Carbide, welches ein Dimethylsiloxan/Methyloctylsiloxan Cyclocopolymer ist. Geeignete flüchtige lineare Silikone weisen 2 bis 9 Si-Atome auf.

Geeignet sind z.B. Hexamethyldisiloxan oder Alkyltrisiloxane wie Hexylheptamethyltrisiloxan oder Octylheptamethyltrisiloxan. Die nicht flüchtigen, hydrophoben Öle haben einen Schmelzpunkt von unterhalb 25°C und einen Siedepunkt von über 250 °C, vorzugsweise über 300 °C. Hierfür kann prinzipiell jedes dem Fachmann allgemein bekannte Öl eingesetzt werden. In Frage kommen pflanzliche oder tierische Öle, Mineralöle, Silikonöle oder deren Mischungen. Geeignete Silikonöle sind Polydimethylsiloxane, phenylierte Silikone, Polyphenylmethylsiloxane, Phenyltrimethicone, Poly(C1-C20)-alkylsiloxane, Alkylmethylsiloxane. Geeignet sind weiterhin Kohlenwasserstofföle, z.B. Paraffin- oder Isoparaffinöle, Squalan, Öle aus Fettsäuren und Polyolen, insbesondere Triglyceride von C10- bis C30-Fettsäuren. Geeignete pflanzliche Öle sind z.B. Sonnenblumenöl, Kokosöl, Rizinusöl, Lanolinöl, Jojobaöl, Maisöl, Sojaöl. Besonders bevorzugt sind Kohlenwasserstofföle, insbesondere Mineralöle (Paraffinum liquidum) sowie pflanzliche Öle und Fettsäuretriglyceride.

Eine Ausführungsform der Erfindung ist ein silikonhaltiges Zweikomponenten-Pflegeshampoo (2-in-1-Shampoo). Silikonshampoos und deren Herstellung werden z.B, in der WO 98/05296 und der darin zitierten Literatur beschrieben. Bei herkömmlichen Silikonshampoos ist eine dauerhaft stabile Dispergierung der unlöslichen Silikone erforderlich, was hohe Anforderungen an das Herstellverfahren zur Einstellung bestimmter Partikelgrößen stellt. Oder es sind Zusatzstoffe zur Stabilisierung erforderlich, z.B. Verdicker, die der Zusammensetzung eine die Auftrennung verhindernde Fließgrenze verleihen. Auf diese Maßnahmen kann erfindungsgemäß verzichtet werden, da es bei einer Dispergierung unmittelbar vor der Anwendung auf eine dauerhafte Stabilität der Dispersion nicht ankommt. Eine der Komponenten des erfindungsgemäßen Zweikomponenten-Shampoos enthält eine wässrige Zusammensetzung mit mindestens einem waschaktiven Tensid, ausgewählt aus anionischen, nichtionischen, zwitterionischen oder amphoteren Tensiden. Die zweite Komponente enthält eine wasserunlösliche flüchtige oder nicht-flüchtige Silikonverbindung entweder als Reinstoff oder in einem geeigneten Lösungsmittel oder als wässrige Voremulsion. Vorzugsweise ist zusätzlich in mindestens einer der beiden Komponenten mindestens ein hierfür bekanntes kationisches Polymer zur Unterstützung der Ablagerung der Silikone auf dem Haar enthalten. Geeignete Tenside, Silikone und kationische Polymere sind neben den oben erwähnten insbesondere die in der WO 98/05296 genannten.

Erfindungsgemäß herstellbare Haarkurzusammensetzungen werden aus einer hydrophilen und einer hydrophoben Komponente gebildet und enthaltend mindestens einen Wirkstoff, ausgewählt aus C10- bis C30-Fettalkoholen, den oben genannten Ölen und den oben genannten kationischen Pflegestoffen. Vorzugsweise handelt es sich bei der fertigen Mischung um eine Fettalkoholdispersion. Die Fettalkohole können in einer Menge von 0,1 - 20 Gew.%, vorzugsweise 0,5 - 10 Gew.%, besonders bevorzugt von 1 bis 8 Gew.% vorliegen. Geeignete Fettalkohole sind primäre Alkohole, insbesondere 1-Alkanole mit 6 bis 26, vorzugweise 12 bis 22 C-Atomen.Insbesondere hat sich die Verwendung von Octanol, Decanol, Dodecanol bzw. Laurinalkohol, Tetradecanol bzw. Myristinalkohol, Hexadecanol bzw. Cetylalkohol, Octadecanol bzw. Stearinalkohol, oder Gemische dieser Fettalkohole, als vorteilhaft herausgestellt. Ein ganz bevorzugter Fettalkohol ist Cetylalkohol. Die Fettalkohole können in einer geeigneten fluiden Zusammensetzung eingesetzt werden, z.B. sofern sie bei Raumtemperatur fest vorliegen, in Form einer Lösung oder Dispersion in einem geeigneten Lösungs- oder Dispersionsmedium, beispielsweise als wässrige Voremulsion. Kationische Pflegestoffe sind die oben genannten und können in der fertigen Mischung in einer Menge von 0,01 bis 10, besonders bevorzugt von 0,05 bis 5 Gew.% enthalten sein.

In einer Ausführungsform handelt es sich um eine cremeförmige, hochviskose Haarkur, die vorzugsweise nach der Anwendung ausgespült wird (Rinse-Produkt). Der Fettalkoholgehalt beträgt vorzugsweise von 0,01 bis 20 Gew.%, besonders bevorzugt von 1 bis 10 Gew.%. Die Viskosität beträgt vorzugsweise von 1000 bis 10000, besonders bevorzugt von 1500 bis 8000 mPa s, gemessen als dynamische Viskositätsmessung mit einem HAAKE Rotationsviskosimeter VT550 bei einer Temperatur von 25°C mit einem Prüfkörper nach DIN 53019(SV-DIN) und einer Schergeschwindigkeit von 50 s⁻¹. Eine weitere Ausführungsform betrifft versprühbare leave-in Haarkuren. Diese bestehen aus einer hydrophilen und einer hydrophoben Phase, welche mittels eines Mikromischers dispergiert werden. Es sind im wesentlichen die gleichen Inhaltsstoffe enthalten wie bei den oben genannten Haarkuren. Der Gehalt an hydrophober Phase ist gegenüber cremeförmigen, auszuspülenden Haarkuren deutlich verringert, so dass sich keine viskosen bzw. flüssigkristallinen Strukturen ausbilden. Die Viskosität ist deutlich niedriger und die Produkte sind versprühbar. Der Fettalkoholgehalt beträgt bei leave-in Produkten vorzugsweise von 0,01 bis 3 Gew.%, besonders bevorzugt von 0,1 bis 1 Gew.%. Die Viskosität von leave-in Produkten beträgt vorzugsweise von 100 bis 2000, besonders bevorzugt von 300 bis 1500 mPa s, gemessen als dynamische Viskositätsmessung mit einem HAAKE Rotationsviskosimeter VT550 bei einer Temperatur von 25°C mit einem Prüfkörper nach DIN 53019(SV-DIN) und einer Schergeschwindigkeit von 50 s⁻¹. Gegenüber herkömmlich hergestellten Sprühkuren ist die Versprühbarkeit deutlich verbessert.

Erfindungsgemäß herstellbare Haarfärbemittel können in einer ersten Komponente mindestens einen haarfärbenden Stoff oder mindestens ein Oxidationsfarbstoffvorprodukt enthalten, welches oxidativ in einen Haarfarbstoff umgewandelt werden kann, und in einer zweiten Komponente mindestens einen Stoff enthalten, ausgewählt aus Oxidationsmitteln, haarpflegenden Stoffen und viskositätserhöhenden Stoffen. Bei den nicht-oxidativen, haarfärbenden Stoffen kann es sich um haarfärbende anorganische Pigmente oder um lösliche, organische, direkt auf das Haar aufziehende Farbstoffe handeln.

Besonders vorteilhaft ist das erfindungsgemäße Verfahren für die Zubereitung von Oxidationsfärbemittel. Oxidationsfärbemittel bestehen in der Regel aus zwei Komponenten: (i) der die Farbstoffvorstufen enthaltenden Farbstoffträgermasse und (ii) der oxidationsmittelzubereitung, die kurz vor dem Gebrauch miteinander vermischt und dann auf das zu färbende Haar aufgetragen werden. Je nach Viskosität und Mischungsverhältnis der beiden Komponenten ergibt sich beim Vermischen eine höhere oder niedrigere Viskosität. Eine gute Haftung des Färbemittels wird hierbei insbeondere durch eine höhere Viskosität des Färbemittels erzielt. Zudem benötigt der Friseur oft für seine Arbeiten höhere Viskositäten, beispielsweise bei speziellen Strähnen- oder Folientechniken sowie um gezielte Arbeiten mit dem Färbepinsel oder dem Akzentuierpinsel verrichten zu können. Mit dem erfindungsgemäßen Verfahren können in einfacher Weise höherviskose Mischungen erzeugt werden mit guten Haftungs- und Färbeeigenschaften.

Als Oxidationsmittel zur Entwicklung der Färbung kommen hauptsächlich Wasserstoffperoxid oder dessen Additionsverbindungen an Harnstoff, Melamin oder Natriumborat in Form einer 1 bis 12%igen, vorzugsweise 1,5 bis 6%igen wässrigen Lösung in Betracht. Das Mischungsverhältnis von Färbemittel zu Oxidationsmittel ist abhängig von der Konzentration des oxidationsmittels und beträgt in der Regel etwa 5:1 bis 1:2, vorzugsweise 1:1, wobei der Gehalt an Oxidationsmittel in der gebrauchsfertigen Mischung vorzugsweise etwa 0,5 bis 8 Gew.%, insbesondere 1 bis 4 Gew.% beträgt.

Die Haarfärbemittel können auf einer emulsionsförmigen Cremebasis beruhen. Bevorzugte Haarfärbemittel weisen einen Gehalt auf an (a) Wasser, (b) mindestens einem bei Raumtemperatur (25°C) festen wachs- oder fettartigen oder bei Raumtemperatur flüssigen, ölförmigen Stoff, (c) mindestens einem Tensid und (d) mindestens einem direktziehenden Haarfarbstoff oder mindestens einer Oxidationsfarbstoffvorstufe- Die Gesamtmenge an Farbstoffen oder Farbstoffvorstufen beträgt vorzugsweise etwa 0,01 bis 10 Gew.%, besonders bevorzugt etwa 0,2 bis 7 Gew.%. Geeignete direktziehende Farbstoffe sind z.B. Triphenylmethanfarbstoffe, aromatische Nitrofarbstoffe, Azofarbstoffe, Chinonfarbstoffe, kationische oder anionische Farbstoffe. Geeignet sind: Nitrofarbstoffe (blau), Nitrofarbstoffe (rot), Nitrofarbstoffe (gelb), basische Farbstoffe, neutrale Azofarbstoffe, saure Farbstoffe.

Als Farbstoffvorstufen können mindestens eine Kupplersubstanz und mindestens eine Entwicklersubstanz eingesetzt werden. Entwickler sind z.B. 1,4-Diamino-benzol (p-Phenylendiamin), 1,4-Diamino-2-methyl-benzol (p-Toluylendiamin), 1,4-Diamino-2-(thiophen-2-yl)benzol, 1,4-Diamino-2-(thiophen-3-yl)benzol, 1,4-Diamino-2-(pyridin-3-yl)benzol, 2,5-Diaminobiphenyl, 1,4-Diamino-2-methoxymethyl-benzol, 1,4-Diamino-2-aminomethyl-benzol, 1,4-Diamino-2-hydroxymethyl-benzol, 4-[Di(2-hydroxyethyl)amino]-anilin, 1,4-Diamino-2-(1-hydroxyethyl)-benzol, 1,4-Diamino-2-(2-hydroxyethyl)-benzol, 1,3-Bis[(4-aminophenyl)(2-hydroxyethyl)amino]-2-propanol, 1,8-Bis(2,5-diaminophenoxy)-3,6-dioxaoctan, 2,5-Diamino-4'-hydroxy-1,1'-biphenyl, 2,5-Diamino-2'-trifluormethyl-1,1'-biphenyl, 2,4',5-Triamino-1,1'-biphenyl, 4-Aminophenol, 4-Amino-3-methyl-phenol, 4-Methylamino-phenol, 4-Amino-2-(aminomethyl)-phenol, 4-Amino-2-[(2-hydroxyethyl)-amino]methyl-phenol, 4-Amino-2-(methoxymethyl)-phenol, 5-Amino-salicylsäure, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamino-1-pentyl-1H-pyrazol, 4,5-Diamino-1-(phenylmethyl)-1H-pyrazol, 4,5-Diamino-1-((4-methoxyphenyl)-methyl-1H-pyrazol, 2-Amino-phenol, 2-Amino-6-methyl-phenol, 2-Amino-5-methyl-phenol, 1,2,4-Trihydroxy-benzol, 2,4-Diaminophenol, 1,4-Dihydroxybenzol, 2-(((4-Aminophenyl)amino)-methyl)-1,4-diaminobenzol.

Kuppler sind z.B. N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-ethoxy-5-methyl-benzol, 2,4-Diamino-1-(2-hydroxyethoxy)-5-methylbenzol, 3-Amino-6-methoxy-2-(methylamino)-pyridin, 3,5-Diamino-2,6-dimethoxy-pyridin, 1,3-Diamino-benzol, 2,4-Diamino-1-(2-hydroxyethoxy)-benzol, 1,3-Diamino-4-(3-hydroxypropoxy)-benzol, 1,3-Diamino-4-(2-methoxyethoxy)-benzol, 1,3-Di(2,4-diaminophenoxy)-propan, 2,6-Bis(2-hydroxyethyl)amino-toluol, 5-Amino-2-methyl-phenol, 5-Amino-4-fluor-2-methyl-phenol, 3-Amino-2,4-dichlor-phenol, 3-Amino-2-chlor-6-methyl-phenol, 3-Amino-phenol, 5-[(2-Hydroxyethyl)amino]-2-methyl-phenol, 2-Amino-3-hydroxypyridin, 2,6-Dihydroxy-3,4-dimethylpyridin, 5-Amino-4-chlor-2-methyl-phenol, 1-Naphthol, 1,5-Dihydroxy-naphthalin, 1,7-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 1,3-Dihydroxy-2-methyl-benzol, 5-[(2-Hydroxyethyl)amino]-1,3-benzodioxol, 3,4-Diamino-benzoesäure, 3,4-Dihydro-6-hydroxy-1,4(2H)-benzoxazin, 3-ethyl-1-phenyl-5-pyrazolon, 5,6-Dihydroxy-indol, 5,6-Dihydroxyindolin, 6-Hydroxy-indol, 2,3-Indolindion.

Zur Haarfärbung bekannte und übliche Farbstoffe, die enthalten sein können, sind z.B. in E. Sagarin, "Cosmetics, Science and Technology", Interscience Publishers Inc., New York (1957), Seiten 503 ff. sowie H. Janistyn, "Handbuch der Kosmetika und Riechstoffe", Band 3 (1973), Seiten 388 ff. und K. Schrader "Grundlagen und Rezepturen der Kosmetika", 2. Auflage (1989), Seiten 782-815 beschrieben.

Geeignete haarfärbende Pigmente sind im Anwendungsmedium praktisch unlösliche Farbmittel und können anorganisch oder organisch sein. Auch anorganisch-organische Mischpigmente sind möglich. Bei den Pigmenten handelt es sich vorzugsweise nicht um Nanopigmente. Die bevorzugte Teilchengröße beträgt 1 bis 200 µm, insbesondere 3 bis 150 µm, besonders bevorzugt 10 bis 100 µm. Bevorzugt sind anorganische Pigmente. Die anorganischen Pigmente können natürlichen Ursprungs sein, beispielsweise hergestellt aus Kreide, Ocker, Umbra, Grünerde, gebranntem Terra di Siena oder Graphit. Bei den Pigmenten kann es sich um Weißpigmente wie z.B. Titandioxid oder Zinkoxid, um Schwarzpigmente wie z.B. Eisenoxidschwarz, Buntpigmente wie z.B. Ultramarin oder Eisenoxidrot, um Glanzpigmente, Metalleffekt-Pigmente, Perlglanzpigmente sowie um Fluoreszenz- oder Phosphoreszenzpigmente handeln, wobei vorzugsweise mindestens ein Pigment ein farbiges, nicht-weißes Pigment ist. Geeignet sind Metalloxide, -hydroxide und -oxidhydrate, Mischphasenpigmente, schwefelhaltige Silicate, Metallsulfide, komplexe Metallcyanide, Metallsulfate, -chromate und -molybdate sowie die Metalle selbst (Bronzepigmente). Geeignet sind insbesondere Titandioxid (CI 77891), schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510), Carmine (Cochineal). Besonders bevorzugt sind Pigmente auf Mica- bzw. Glimmerbasis welche mit einem Metalloxid oder einem Metalloxychlorid wie Titandioxid oder Wismutoxychlorid sowie gegebenenfalls weiteren farbgebenden Stoffen wie Eisenoxiden, Eisenblau, Ultramarine, Carmine etc. beschichtet sind und wobei die Farbe durch Variation der Schichtdicke bestimmt ist. Derartige Pigmente werden beispielsweise unter den Handelsbezeichnung Rona^{®}, Colorona^{®}, Dichrona^{®} und Timirori^{®} von der Firma Merck, Deutschland vertrieben. Organische Pigmente sind z.B. die natürlichen Pigmente Sepia, Gummigutt, Knochenkohle, Kasseler Braun, Indigo, Chlorophyll und andere Pflanzenpigmente. Synthetische organische Pigmente sind z.B. AzoPigmente, Anthrachinoide, Indigoide, Dioxazin-, Chinacridon-, Phtalocyanin-, Isoindolinon-, Perylen- und Perinon-, Metallkomplex-, Alkaliblau- und Diketopyrrolopyrrolpigmente.

Der pH-Wert des erfindungsgemäßen Färbemittels liegt bei nicht-oxidativen Färbemitteln auf der Basis von direktziehenden Farbstoffen im Bereich von etwa 5 bis 10, vorzugsweise 6 bis 9, während bei oxidativen Färbemitteln auf der Basis von Oxidationsfarbstoffvorstufen der pH-Wert in einem Bereich von etwa 6 bis 12, vorzugsweise 9 bis 11, liegt, wobei der pH-Wert des gebrauchsfertigen Oxidationshaarfärbemittels (das heißt der Mischung des erfindungsgemäßen Haarfärbemittels mit dem Oxidationsmittel) etwa 5,5 bis 10, vorzugsweise 6 bis 9, beträgt. Je nach Zusammensetzung und gewünschtem pH-Wert des Färbemittels erfolgt die Einstellung des pH-Wertes vorzugsweise mit Ammoniak oder organischen Aminen, wie zum Beispiel Glucaminen, Aminomethyl-propanol, Monoethanolamin oder Triethanolamin, anorganischen Basen, beispielsweise Natriumhydroxid, Kaliumhydroxid, Natriumcarbonat oder Calciumhydroxid, beziehungsweise organischen oder anorganischen Säuren, wie z.B. Milchsäure, Zitronensäure, Essigsäure oder Phosphorsäure.

Nach dem erfindungsgemäßen Verfahren können auch kosmetische Sonnenschutzmittel unmittelbar vor der Anwendung hergestellt werden, wobei die Mischung mindestens einen Lichtschutzwirkstoff enthält. Besonders bevorzugt sind disperse Sonnenschutzmittel, welche entweder unlösliche Lichtschutzwirkstoffe in fein dispergierter Form enthalten oder disperse Sonnenschutzmittel, welche aus einer Öl- oder Lipidphase und einer wässrigen Phase bestehen. Hierbei kann es sich um O/W- oder W/O-Emulsionen handeln. Herkömmliche Sonnencremes sind schwer zu stabilisieren, um die hohen Anforderungen an eine langfristige Stabilität zu erfüllen und es ist eine auswahl speziell abgestimmter Emulgatorgemische erforderlich. Erfindungsgemäße ZweiKomponenten-Sonnenschutzmittel, bei welchem die Dispergierung unmittelbar vor der Anwendung erfolgt, haben den Vorteil, dass die Anforderungen an das Emulgatorsystem wesentlich geringer sind, andere, insbesondere hautfreundlichere Emulgatoren eingesetzt werden können, die Emulgatormenge reduziert oder sogar ganz oder teilweise auf Emulgatoren verzichtet werden kann. Der Lichtschutzwirkstoff kann ausgewählt sein aus UV-Licht absorbierenden anorganischen Pigmenten, anorganischen Nanopigmenten und öl- oder wasserlöslichen organischen WA-, UVB- oder UVA/UVB-Filtersubstanzen. Geeignete Filtersubstanzen sind z.B. 2-Phenylbenzimidazol-5-sulphonsäure und deren Salze, Zimtsäurederivate, Salicylsäurederivate, Campherderivate, Triazinderivate, Benzophenonderivate, Dibenzoylmethanderivate, ß,ß-Diphenylacrylatderivate, p-Aminobenzoesäurederivate, Menthylanthranilat, Polymere mit Lichtschutzwirkung und Silikone mit Lichtschutzwirkung. Die erfindungsgemäß hergestellten Sonnenschutzmittel können sich durch einen verbesserten Lichtschutzfaktor auszeichnen.

Nach dem erfindungsgemäßen Verfahren können auch kosmetische, dermatologische oder pharmazeutische Hautcremes unmittelbar vor der Anwendung hergestellt werden , wobei das Produkt eine aus einer wässrigen Phase und einer hydrophoben Phase aufgebaute Emulsion ist, mindestens einen hautpflegenden, dermatologischen oder pharmazeutischen Wirkstoff enthält und die Dispergierung der Phasen in einem Mikromischer erfolgt. Die Hautcreme enthält in der Regel Wasser, einen Fett- oder Wachsstoff, einen Emulgator und einen Wirkstsoff. Bei dem Wirkstoff kann es sich um kosmetische Öle, Emollients, Vitamine, Vitaminderivate, Provitamine, essentielle Fettsäuren, Sphingolipide, Phospholipide, Ceramide, Betain, Panthenol, Pharmazeutika etc. handeln. Erfindungsgemäß hergestellte Hautcremes zeichnen sich durch ein verbessertes Hautgefühl, eine verbesserte Verteilung der Wirkstoffe, ein verbessertes Aufziehen der Wirkstoffe auf die Haut und eine Verringerung der benötigten Einsatzmenge aus. Außerdem kann die Emulgatormenge verringert werden, was die Gefahr von Hautreizungen und Hautirritationen verringert.

Nach dem erfindungsgemäßen Verfahren können auch haar- oder hautkosmetischen Zubereitungen hergestellt werden, welche mindestens einen pulverförmigen Feststoff in fein dispergierter Form enthalten, wobei die Dispergierung des Feststoffs in einem Mikromischer erfolgt. Geeignete Feststoffe sind z.B. Pigmente, Perlglanzmittel, Talk, Glimmer, Kaolin, Zinkoxide, Titanoxide, gefälltes Calciumcarbonat, Magnesiumcarbonat oder -hydrogencarbonat, Kieselsäure, Glaskugeln, keramische Kugeln, pulverförmige Polymere etc. Vorzusgweise liegen die Feststoffe in einer geeigneten Vorsuspension vor.

Nach dem erfindungsgemäßen Verfahren können auch Parfümöle und Duftstoffe enthaltende Wirkstoffzubereitungen unmittelbar vor der Anwendung hergestellt werden. Eine erste Komponente enthält dabei eine unparfümierte Wirkstoffzusammensetzung und eine zweite Komponente enthält mindestens ein Parfümöl oder Duftstoff. Hierdurch wird ermöglicht, Arten und Mengen von Duftstoffen einzusetzen, die ansonsten nicht über längere Zeit in Kombination mit der Wirkstoffzubereitung stabil wären. Außerdem ist es für den Anwender möglich, bei Verwendung von austauschbaren Verpackungsteilen für die einzelnen Komponenten unterschiedliche Wirkstoffzusammensetzungen mit unterschiedlichen Parfümierungen individuell zu kombinieren.

Die Komponenten können, soweit nicht bereits genannt, weitere Wirk- und Zusatzstoffe enthalten. Als Wirk- und Zusatzstoffe kommen beispielsweise in Frage: weitere waschaktive anionische, nichtionisch oder amphotere Tenside, Antischuppenwirkstoffe, Haar- und Hautpflegestoffe wie quaternisierte Alkylamine, kationische Polymere natürlichen oder synthetischen Ursprungs, Proteine und ihre Derivate, wie z.B. Kollagen-, Keratin-, Seidenprotein- und Weizenproteinhydrolysate sowie Silikonverbindungen. Desweiteren können eingesetzt werden: Parfümöle, Farbstoffe, Trübungsmittel wie z.B. Glycoldistearat; haarkonditionierende Mittel wie synthetische oder natürliche Phospholipide oder quaternäre Derivate der Stärke oder Cellulose; Lösungsvermittler wie kurzkettige Alkohole, z.B. Ethanol, n-Propanol, Isopropanol oder Glykole wie Butylen- oder Propylenglykol; Aminosäuren wie z.B. Histidin, Glycin, Alanin, Threonin, Arginin, Cystein und deren Derivate wie z.B. Fettsäurekondensationsprodukte oder quaternäre Produkte; weitere Wirkstoffe wie Pflanzenextrakte, Vitamine, Allantoin, Chitosan, Konservierungsmittel etc..

Die Vorteile der erfindungsgemäß hergestellten vermischten Produkte bestehen in einer optimalen Teilchengrößenverteilung einhomogenisierter Teilchen, einer optimalen Verteilung der dispersen Phase in der externen Phase, einer hohen wirksamen Oberfläche, einem reduzierten Bedarf an Emulgatormenge und dadurch bedingt einer verbesserten Hautverträglichkeit, einer verbesserten Wirksamkeit kosmetischer Wirk- und Hilfsstoffe, einem verbesserten Kristallisationsverhalten und verbesserten rheologischen Eigenschaften. Erfindungsgemäß hergestellte Haar- und Hautbehandlungsmittel weisen den Vorteil auf, dass sie eine gleichmäßigere Ablagerung von Wirkstoffen auf dem Haar oder auf der Haut ermöglichen als herkömmlich hergestellte Produkte. Die engere Teilchengrößenverteilung verbessert das Aufziehen auf das Haar.

### Beispiele

Die folgenden Rezepturbeispiele können in Kombination mit einer erfindungsgemäßen Verpackungseinheit verwendet werden.
xxxxxx

### Beispiel 1: Haarstylinggel aus zwei dünnflüssigen Phasen

### Komponente 1:

| | |
|---|---|
| 0,5 g | Carbomer (vernetzte Polyacrylsäure, Carbopol^{®} 980) |
| 40 g | Wasser |

### Komponente 2:

| | |
|---|---|
| 2 g | Polyvinylpyrrolidon (PVP K90) |
| 3 g | Glycerin |
| 0,4 g | Aminomethylpropanol |
| 0,4 g | PEG-40 HYDROGENATED CASTOR OIL (Cremophor^{®} CO410 |
| 0,2 g | Parfüm |
| 15 g | Ethanol |
| ad 50 g | Wasser |

### Beispiel 2: Oxidatives Haarfärbemittel

### Komponente 1:

| | |
|---|---|
| 17 g | Cetearylalkohol |
| 1,9 g | Natriumlaurylsulfat |
| 1,4 g | Natriumlaurylethersulfat |
| 2,1 g | Lanolinalkohol |
| 6,1 g | Glycerylstearat |
| 0,4 g | Natrium Cocoylisoethionat |
| 1,4 g | Ammoniak |
| 6 g | Isopropanol |
| 0,6 g | Natriumsulfit |
| 0,3 g | EDTA |
| 0,06 g | p-Aminophenolhydrochlorid |
| 0,65 g | p-Toluylendiaminsulfat |
| 0,26 g | Resorcinol |
| 0,3 g | Parfümöl |
| ad 100 g | Wasser |

### Komponente 2:

6%ige Wasersoffperoxidlösung;
oder Wasserstoffperoxid-Emulsion:

| | |
|---|---|
| 10,0 g | Cetylstearylalkohol |
| 1,5 g | Cholesterin |
| 4,0 g | Natriumlaurylalkoholdiglykolethersulfat, 28 %ige wässrige Lösung |
| 35,0 g | Wasserstoffperoxid, 35 %ige wässrige Lösung |
| 0,3 g | Parfüm |
| ad 100,0 g | Wasser |
| | |

### Beispiel 3: Shampoo

### Komponente 1:

| | |
|---|---|
| 30 g | Natriumlaurylethersulfat |
| 8 g | Cocamidopropylbetain |
| 3 g | Glykoldistearat |
| 0,35 g | Natriumbenzoat |
| 0,15 g | Natriumformiat |
| 0,2 g | Natriumchlorid |
| ad 100 g | Wasser |

### Komponente 2:

Kationisches Polymer und/oder Parfüm und/oder Silikonöl in geeignetem Lösungsmittel oder als wässrige Voremulsion.

**Beispiel 4: Sonnenschutzmittel**

| **Ölphase:** | **INCI/ EU** | **Gew.%** |
|---|---|---|
| Parsol 1789 | BUTYL METHOXYDIBENZOYLMETHANE | 0,30 |
| Neo Heliopan AV/OA | OCTYL METHOXYCINNAMATE | 10,00 |
| Hostaphat KL 340 N | TRILAURETH-4 PHOSPHATE | 0,60 |
| Hostacerin DGI | POLYGLYCERYL-2 SESQUIISOSTEARATE | 0,70 |
| Phenoxetol | PHENOXYETHANOL | 1,00 |
| Cetiol 868 | OCTYL STEARATE | 5,00 |
| Primol 352 | MINERAL OIL | 5,00 |
| Abil Wax 9801D | CETYL DIMETHICONE | ---- |
| **Wasserphase:** | | |
| Carbopol 2984 | CARBOMER | 0,30 |
| Natriumhydroxid | SODIUM HYDROXIDE | 0,06 |
| Glycerin 86% | GLYCERIN | 5,00 |
| Wasser, vollentsalzt | AQUA | Ad 100 |

**Beispiel 5: Sonnenschutzmittel**

| **Ölphase:** | **INCI/ EU** | **Gew. %** |
|---|---|---|
| Parsol 1789 | BUTYL METHOXYDIBENZOYLMETHANE | 0,30 |
| Neo Heliopan AV/OA | OCTYL METHOXYCINNAMATE | 10,00 |
| Hostaphat KL 340 N | TRILAURETH-4 PHOSPHATE | 0,60 |
| Hostacerin DGI | POLYGLYCERYL-2 SESQUIISOSTEARATE | 0,70 |
| Phenoxetol | PHENOXYETHANOL | 1,00 |
| Cetiol 868 | OCTYL STEARATE | 9,00 |
| Primol 352 | MINERAL OIL | --- |
| Abil Wax 9801D | CETYL DIMETHICONE | 1,00 |
| **Wasserphase:** | | |
| Carbopol 2984 | CARBOMER | 0,30 |
| Natriumhydroxid | SODIUM HYDROXIDE | 0,06 |
| Glycerin 86% | GLYCERIN | 5,00 |
| Wasser, vollentsalzt | AQUA | Ad 100 |

**Beispiel 6: Sonnenschutzmittel**

| **Lipidphase:** | **INCI/ EU** | **Gew. %** |
|---|---|---|
| Parsol 1789 | BUTYL METHOXYDIBENZOYLMETHANE | 1,50 |
| PHB- Methylester | METHYLPARABEN | 0,20 |
| Neo Heliopan AV/OA | OCTYL METHOXYCINNAMATE | 10,00 |
| Neo Heliopan Type 303 | OCTOCRYLENE | 10,00 |
| Finsolv TN | C12-15 ALKYL BENZOATE | 2,50 |
| Eutanol G | OCTYLDODECANOL | 10,00 |
| Antaron V 216 | PVP/HEXADECENE COPOLYMER | 2,00 |
| Vitamin E- Acetat | TOCOPHERYL ACETATE | 0,50 |
| Parfum | PARFUM | 0,30 |
| Abil Wax 9801D | CETYL DIMETHICONE | 0,50 |
| **Wasserphase:** | | |
| Carbopol 1382 | ACRYLATES/C10-30 ALKYL ACRYLATE CROSSPOLYMER | 0,45 |
| Colorona Oriental Beige 17237 | MICA (and) CI 77891 (and) CI 77491 | 0,05 |
| Glycerin 86% | GLYCERIN | 5,00 |
| Edeta BD | DISODIUM EDTA | 0,10 |
| Natriumhydroxid | SODIUM HYDROXIDE | 0,18 |
| D-Panthenol | PANTHENOL | 0,50 |
| Wasser, vollentsalzt | AQUA | Ad 100 |
| Dekaben LMB | IODOPROPYNYL BUTYLCARBAMATE | 0,50 |

**Beispiel 7: O/W-Körperlotion**

| **Ölphase:** | **INCI/ EU** | **Gew. %** |
|---|---|---|
| Paraffinöl subliquidum | PARAFFINUM LIQUIDUM | 7,50 |
| Cetagol V | CETEARYL ISONONANOATE | 2,50 |
| Avocadoöl | PERSEA GRATISSIMA | 2,00 |
| Hostaphat 340 D | TRILAURETH-4 PHOSPHATE | 3,50 |
| Methylparaben | METHYLPARABEN | 0,20 |
| Propylparaben | PROPYLPARABEN | 0,05 |
| Parfum | PARFUM | 0,50 |
| **Wasserphase:** | | |
| Carbopol 2984 | CARBOMER | 0,30 |
| Natriumhydroxid | SODIUM HYDROXIDE | 0,06 |
| Phenoxetol | PHENOXYETHANOL | 0,60 |
| Glycerin 86% | GLYCERIN | 5,00 |
| Wasser, vollentsalzt | AQUA | Ad 100 |

**Beispiel 8: W/O-Körperlotion**

| **Ölphase:** | **INCI/ EU** | **Gew. %** |
|---|---|---|
| Paraffinöl subliquidum | PARAFFINUM LIQUIDUM | 15,00 |
| Cetiol 868 | ETHYLHEXYL STEARATE | 12,00 |
| Dehymuls PGPH | POLYGLYCERYL-2 DIPOLYHYDROXYSTEARATE | 2,00 |
| Zincum N29 | ZINC STEARATE | 0,50 |
| Vitamin E - Acetat | TOCOPHERYL ACETATE | 0,50 |
| Methylparaben | METHYLPARABEN | 0,20 |
| Propylparaben | PROPYLPARABEN | 0,05 |
| Parfum | PARFUM | 0,50 |
| **Wasserphase:** | | |
| Zinksulfat 7-Hydrat | ZINC SULFATE | |
| Phenoxetol | PHENOXYETHANOL | 0,60 |
| Glycerin 86% | GLYCERIN | 8,00 |
| Wasser, vollentsalzt | AQUA | Ad 100 |

### Bezugszeichenliste

1 Platte
2 Eintrittsöffnung
3 Verbindungskanal
4 Austrittsöffnung
5 Mischzone
6 Mikrostruktureinheit
7 Teilkanal
8 Aussparung
9 Durchtrittsöffnung
10 Einbauten
11 Gehäuse
12 Durchbruch
12a Fluidzuführung
13 zusätzlicher Teilkanal
13a Verschlußvorrichtung
14 Halterungselement
15 Deckel
16 Fluidabführung
17 Außenbehälter
18a,b Innenbehälter

## Patentansprüche

1. Verpackungssystem mit mindestens zwei separaten Vorratskammern zur in situ Herstellung von Formulierungen aus mindestens zwei bis zur Anwendung voneinander getrennt gehaltenen Komponenten, wobei es mindestens einen statischen Mikromischer aufweist, **dadurch gekennzeichnet, dass** der Mikromischer mindestens ein Bauteil in Form einer Platte (1) aufweist und wobei die Platte (1)
- mindestens eine Eintrittsöffnung (2) für den Eintritt mindestens eines Eduktstroms in einen in der Plattenebene liegenden Verbindungskanal (3) und mindestens eine Austrittsöffnung (4) für den Austritt des Eduktstroms in eine in der Plattenebene liegende Mischzone (5) aufweist,
- wobei die Eintrittsöffnung (2) mit den Austrittsöffnungen (4) durch den in der Plattenebene liegenden Verbindungskanal (3) kommunizierend verbunden ist und
- wobei der Verbindungskanal (3) vor der Mündung in die Mischzone (5) durch Mikrostruktureinheiten (6) in zwei oder mehr Teilkanäle (7) aufgespalten wird, wobei die Breiten der Teilkanäle im Millimeter- bis Submillimeterbereich liegen und kleiner sind als die Breite der Mischzone (5),
und wobei das Verhältnis der größten Breite des Verbindungskanals (3) zur Breite der Teilkanäle (7) an deren Austritt in die Mischzone (5) größer 2 ist.

2. Verpackungssystem nach Anspruch 1, **dadurch gekennzeichnet, dass** es eine Vorrichtung zur Förderung der getrennt gehaltenenen Komponenten durch den Mikromischer aufweist und der Mikromischer ein Gehäuse mit mindestens 2 Eduktzuführungen und mindestens einer Produktabführung aufweist.

3. Verpackungssystem nach Anspruch 1 oder 2, **dadurch gekennzeichnet, dass** der Mikromischer zwei oder mehr im Gehäuse zu einem Stapel angeordnete Platten (1) aufweist, wobei die Platten (1) so übereinanderliegen, dass die Eintrittsöffnungen (2) mit den Eduktzuführungen verbundene. Nebenkanäle zum Zuführen des jeweiligen zu vermischenden Edukts und die Mischzonen (5) zusammen einen mit der Produktabführung verbundenen Hauptkanal zum Abführen des vermischten Produktes bilden und sich die Haupt- und Nebenkanäle durch den Stapel erstrecken.

4. Verpackungssystem nach einem der vorhergehenden Ansprüche, **dadurch gekennzeichnet, dass** die Breiten der Teilkanäle (7) der Platten (1) an der Mündung in die Mischzone (5) 1 µm bis 2 mm betragen;
und/oder das Verhältnis der größten Breite des Verbindungskanals (3) und/oder der Breite der Eintrittsöffnung (2) zur Breite der Teilkanäle (7) der Platten (1) größer 5 ist;
und/oder das Verhältnis der Länge zur Breite der Teilkanäle (7) der Platten (1) von 1:1 bis 20:1 beträgt; und/oder das Verhältnis der Breite der Mischzone (5) zur Breite der Teilkanäle(7) der Platten (1) größer 2 ist.

5. Verpackungssystem nach einem der vorhergehenden Ansprüche, **dadurch gekennzeichnet, dass** die Platte (1) zusätzlich mindestens eine Durchtrittsöffnung (9) aufweist.

6. Verpackungssystem nach einem der vorhergehenden Ansprüche, **dadurch gekennzeichnet, dass** mindestens eine der Eintritts- (2) oder Durchtrittsöffnungen (9) oder die Mischzone (5) der Platte (1) von der Plattenebene umschlossen vorliegt und der Verbindungskanal (3) durch eine Vertiefung ausgebildet ist.

7. Verpackungssystem nach einem der vorhergehenden Ansprüche, **dadurch gekennzeichnet, dass** mindestens eine der Eintritts- (2) oder Durchtrittsöffnungen (9) oder die Mischzone (5) der Platte (1) am Plattenrand oder durch Ausparungen am Plattenrand angeordnet ist.

8. Verpackungssystem nach einem der vorhergehenden Ansprüche, **dadurch gekennzeichnet, dass** die Platte (1) mindestens zwei Eintrittsöffnungen (2) für mindestens zwei verschiedene Fluidströme aufweist, wobei jede Eintrittsöffnung (2) durch je einen Verbindungskanal (3) mit der Mischzone (5) verbunden ist.

9. Verpackungssystem nach einem der vorhergehenden Ansprüche, **dadurch gekennzeichnet, dass** die Austrittsöffnungen (4) der Platte (1) auf einer kreisförmigen Linie angeordnet sind.

10. Verpackungssystem nach einem der vorhergehenden Ansprüche, **dadurch gekennzeichnet, dass** die Platte (1) zusätzliche Durchbrüche (12) und in die Mikrostruktureinheiten (6) integrierte, von den Teilkanälen (7) getrennte, zusätzliche Teilkanäle (13) aufweist.

11. Verpackungssystem nach einem der vorhergehenden Ansprüche, **dadurch gekennzeichnet, dass** entweder die Verbindungskanäle (3) der Platten (1) durch Vertiefungen ausgebildet sind und die Verbindungskanäle (3) vor der Mündung in die Mischzone (5) durch auf den Platten (1) angebrachten Mikrostruktureinheiten (6) in Teilkanäle (7) aufgespalten werden oder dass die Verbindungskanäle (3) der Platten (1) durch Ausnehmungen in den Platten (1) gebildet sind, wobei die Platten als Zwischenplatten zwischen je einer Deck- und einer Bodenplatte angeordnet sind und die Verbindungskanäle (3) vor der Mündung in die Mischzone (5) durch an den Deck- und/oder Bodenplatten angebrachten Mikrostruktureinheiten (6) in Teilkanäle (7) aufgespalten werden.

12. Verpackungssystem nach einem der vorhergehenden Ansprüche, **dadurch gekennzeichnet, dass** die Mischzone (5) in Ruhestellung durch einen die Austrittsöffnungen (4) verschließenden Formkörper ausgefüllt ist, welcher bei Betrieb ganz oder teilweise aus der Mischzone (5) entfernbar ist und die Austrittsöffnungen (4) ganz oder teilweise freigibt.

13. Verfahren zur in situ Herstellung von aus mindestens zwei Komponenten bestehenden Formulierungen, wobei die in separaten Vorratsbehältern vorliegenden Komponenten unmittelbar vor der Anwendung der Formulierung miteinander mittels eines Verpackungssystems gemäß einem der Ansprüche 1 bis 13 vermischt werden.

14. Verfahren nach Anspruch 13, **dadurch gekennzeichnet, dass** die Einströmungsgeschwindigkeit des Eduktstroms in die Mischzone (5) größer ist als die Strömungsgeschwindigkeit des Produktstroms innerhalb der Mischzone (5).

15. Verfahren nach einem der Ansprüche 13 bis 14, **dadurch gekennzeichnet, dass** es sich bei der Formulierung um eine Mikro- oder Nanoemulsion handelt.

16. Verfahren nach einem der Ansprüche 13 bis 15, **dadurch gekennzeichnet, dass** die eine Komponente eine wässrige, flüssige Phase und die andere Komponente eine hydrophobe, flüssige oder eine einen wasserempfindlichen Stoff enthaltende Phase ist; oder dass die Komponenten Stoffe enthalten, die bei Kontakt miteinander chemisch reagieren oder die physikalische Konsistenz der Mischung verändern.

17. Verfahren nach einem der Ansprüche 13 bis 16 zur Herstellung von Färbemitteln, Klebemitteln, Lebensmitteln, pharmazeutischen Mitteln, kosmetischen Mitteln, Baustoffen oder Reinigungsmitteln.

18. Verfahren nach Anspruch 17 zur Herstellung von emulsionsförmigen, mindestens einen haar- oder hautpflegenden kosmetischen, dermatologischen oder pharmazeutischen Wirkstoff enthaltenden Präparaten, haarfestigenden Mitteln, Haarfärbemitteln oder Dauerwellmitteln.

## Claims

1. Packaging system containing at least two separate storage compartments for the *in situ* production of formulations consisting of at least two components to be used that are held separate from one another, wherein at least one has a static micromixer, **characterized in that** the micromixer has at least one component in the form of a plate (1) and wherein the plate (1)
- has at least one inlet aperture (2) for the supply of at least one stream of starting material into a connection channel (3) lying at the plate level, and at least one outlet aperture (4) for the discharge of the stream of starting material into a mixed zone (5) lying at the plate level,
- wherein the inlet aperture (2) is connected in an interactive way with the outlet aperture (4) by the connection channel (3) lying at the plate level, and
- wherein in front of the opening in the mixed zone (5), the connection channel (3) is split by micro-structural units (6) into two or more sub-channels (7), wherein the widths of the sub-channels range from millimeters to sub-millimeters and are smaller than the width of the mixed zone (5),
and wherein the ratio of the greatest width of the connection channel (3) to the widths of the sub-channels (7) at their discharge into the mixed zone (5) is greater than 2.

2. Packaging system according to Claim 1, **characterized in that** it has a device to convey the components held separately through the micromixer, and the micromixer has a housing containing at least 2 streams of starting material and at least one product discharge.

3. Packaging system according to Claim 1 or 2, **characterized in that** the micromixer has two or more plates (1) arranged in the housing in a stack, wherein the plates (1) lie on top of one another so that the inlet apertures (2) form side channels connected to the supplies of starting material to feed the respective stream of starting material to be mixed and the mixed zones (5) together form a main channel connected to the product discharge to discharge the mixed product, and the main and side channels extend through the stack.

4. Packaging system according to one of the preceding claims, **characterized in that** the widths of the sub-channels (7) of the plates (1) at the opening into the mixed zone (5) are 1 µm to 2 mm;
and/or the ratio of the greatest width of the connection channel (3) and/or the width of the inlet aperture (2) to the widths of the sub-channels (7) of the plates (1) is greater than 5;
and/or the ratio of the length to the width of the sub-channels (7) of the plates (1) is from 1:1 to 20:1;
and/or the ratio of the width of the mixed zone (5) to the widths of the sub-channels (7) of the plates (1) is greater than 2.

5. Packaging system according to one of the preceding claims, **characterized in that** the plate (1) also has at least one passage opening (9).

6. Packaging system according to one of the preceding claims, **characterized in that** at least one of the inlet apertures (2) or passage openings (9) or the mixed zone (5) of the plate (1) is closed at the plate level and the connection channel (3) is formed by a depression.

7. Packaging system according to one of the preceding claims, **characterized in that** at least one of the inlet apertures (2) or passage openings (9) or the mixed zone (5) of the plate (1) is arranged on the edge of the plate or by recesses on the edge of the plate.

8. Packaging system according to one of the preceding claims, **characterized in that** the plate (1) has at least two inlet apertures (2) for at least two different streams of fluid, wherein each inlet aperture (2) is connected to the mixed zone (5) in each instance by a connection channel (3).

9. Packaging system according to one of the preceding claims, **characterized in that** the outlet apertures (4) of the plate (1) are arranged on a circular line.

10. Packaging system according to one of the preceding claims, **characterized in that** the plate (1) has additional perforations (12) and additional sub-channels (13) integrated into the micro-structural units (6) and separated by the sub-channels (7).

11. Packaging system according to one of the preceding claims, **characterized in that** either the connection channels (3) of the plates (1) are formed by depressions and in front of the opening in the mixed zone (5), the connection channels (3) are split by micro-structural units (6) attached to the plates (1) into sub-channels (7) or that the connection channels (3) of the plates (1) are formed by recesses in the plates (1), wherein the plates in each instance are arranged as intermediate plates between a cover plate and a base plate, and in front of the opening in the mixed zone (5), the connection channels (3) are split by micro-structural units (6) attached to the cover and/or base plates into sub-channels (7).

12. Packaging system according to one of the preceding claims, **characterized in that** the mixed zone (5) is filled out in the rest position by a shaped body that blocks the outlet apertures (4) and which can be removed, in whole or in part, from the mixed zone (5) during operation, and which unblocks some or all of the outlet apertures (4).

13. Method for the *in situ* production of formulations consisting of at least two components, wherein the components located in separate storage compartments are mixed together immediately before use of the formulation by means of a packaging system according to one of Claims 1 through 13.

14. Method according to Claim 13, **characterized in that** the speed of the inflowing stream of starting material into the mixed zone (5) is greater than the streaming speed of the product stream within the mixed zone (5).

15. Method according to Claims 13 or 14, **characterized in that** the formulation is a micro- or nano-emulsion.

16. Method according to one of Claims 13 to 15, **characterized in that** the phase containing a component is a liquid aqueous phase and the other component is a hydrophobic liquid or a water-sensitive substance; or that the components contain substances that chemically react upon contact with one another or the components change the physical consistency of the mixture.

17. Method according to one of Claims 13 to 16 to produce dyes, adhesives, food, pharmaceuticals, cosmetics, building materials or cleaning agents.

18. Method according to Claim 17 to produce preparations in the form of emulsions that contain at least one hair-care or skin-care cosmetic, dermatological or pharmaceutical active ingredient, hair-setting agents, hair-coloring agents or permanent-wave agents.

## Revendications

1. Système d'emballage avec au moins deux chambres d'approvisionnement séparées pour la réalisation in situ de formulations d'au moins deux composants conservés séparément jusqu'à l'emploi, en même temps, il comporte au moins un micro mélangeur statique, **caractérisé en ce que** le micro mélangeur comprend dans sa construction au moins une partie en forme de plateau (1), et où le plateau (1)
- présente au moins un orifice d'entrée (2) pour l'approvisionnement d'au moins un flux de réactif dans un canal de liaison (3) situé sur la partie plane du plateau et au moins un orifice de sortie (4) pour l'écoulement du flux de réactif dans une zone de mélange (5) située sur la partie plane du plateau,
- en même temps, l'orifice d'entrée (2) est communiquant avec l'orifice de sortie (4) par le canal de liaison (3) situé dans la partie plane du plateau et
- en même temps, le canal de liaison (3) est subdivisé avant de déboucher dans la zone de mélange (5) par des unités micro structurelles (6) en deux ou plusieurs canaux partiels (7), les largeurs des canaux partiels se situent dans les domaines millimétriques à décimillimétriques et sont plus petits que la largeur de la zone de mélange (5),
et, en même temps, le rapport de la largeur la plus grande du canal de liaison (3) sur la largeur des canaux partiels (7) à leur sortie dans la zone de mélange (5) est supérieur à 2.

2. Système d'emballage selon la revendication 1, **caractérisé en ce qu'**il comporte un dispositif pour l'extraction par le micro mélangeur des composants conservés séparément et que le micro mélangeur comporte un boîtier avec au moins deux entrées de réactifs et au moins une sortie de produit.

3. Système d'emballage selon la revendication 1 ou 2, **caractérisé en ce que** le micro mélangeur comporte deux ou plusieurs plateaux (1) empilés dans le boîtier, où les plateaux (1) sont agencés les uns au-dessus des autres de manière à ce que les orifices d'entrée (2), avec les canaux additionnels correspondants aux entrées de réactifs pour l'apport des réactifs respectifs à mélanger et la zone de mélange (5), forment ensemble un canal principal relié à l'évacuation du produit pour l'évacuation du produit mélangé et que les canaux principal et additionnels s'étendent à travers l'empilement.

4. Système d'emballage selon l'une des revendications précédentes, **caractérisé en ce que** les largeurs des canaux partiels (7) des plateaux (1) sont de 1 µm à 2 mm à leur embouchure dans la zone de mélange (5) ;
et/ou que le rapport de la largeur la plus grande des canaux de liaison (3) et/ou de la largeur de l'orifice d'entrée (2) sur la largeur des canaux partiels (7) des plateaux (1) est supérieur à 5 ;
et/ou que le rapport de la longueur sur la largeur des canaux partiels (7) des plateaux (1) est de 1:1 à 20:1 ;
et/ou que le rapport de la largeur de la zone de mélange (5) sur la largeur des canaux partiels (7) des plateaux (1) est supérieur à 2.

5. Système d'emballage selon l'une des revendications précédentes, **caractérisé en ce que** le plateau (1) présente au moins un orifice de passage supplémentaire (9).

6. Système d'emballage selon l'une des revendications précédentes, **caractérisé en ce qu'**au moins l'un des orifices d'entrée (2) ou de passage (9) ou la zone de mélange (5) du plateau (1) se trouve inclus dans la partie plane du plateau et que le canal de liaison (3) est formé par un creux.

7. Système d'emballage selon l'une des revendications précédentes, **caractérisé en ce qu'**au moins l'un des orifices d'entrée (2) ou de passage (9) ou la zone de mélange (5) du plateau (1) se trouve agencé sur le bord du plateau, ou se trouve apparié sur le bord du plateau.

8. Système d'emballage selon l'une des revendications précédentes, **caractérisé en ce que** le plateau (1) comporte au moins deux orifices d'entrée (2) pour au moins deux flux de fluides différents, pendant que chaque orifice d'entrée (2) est relié à la zone de mélange (5) par un des canaux de liaison (3).

9. Système d'emballage selon l'une des revendications précédentes, **caractérisé en ce que** les orifices de sortie (4) du plateau (1) sont agencés en une ligne circulaire.

10. Système d'emballage selon l'une des revendications précédentes, **caractérisé en ce que** le plateau (1) comporte des perforations supplémentaires (12) et des canaux partiels supplémentaires (13) intégrés dans les unités micro structurelles (6), séparées des canaux partiels (7).

11. Système d'emballage selon l'une des revendications précédentes, **caractérisé en ce que**, soit les canaux de liaison (3) des plateaux (1) sont formés par des creux et que les canaux de liaison (3) sont divisés en canaux partiels (7) dans les unités micro structurelles (6) rapportées sur les plateaux (1) avant de déboucher dans la zone de mélange (5), soit les canaux de liaison (3) des plateaux (1) sont formés par des évidements dans les plateaux (1), de ce fait les plateaux sont agencés comme des plateaux intermédiaires entre respectivement un plateau de dessus et un plateau de dessous, et les canaux de liaison (3), avant de déboucher dans la zone de mélange (5), sont subdivisés par des unités micro structurelles (6) rapportées entre des plateaux de dessus et/ou des plateaux de dessous.

12. Système d'emballage selon l'une des revendications précédentes, **caractérisé en ce que** la zone de mélange (5) au repos se trouve fermée par un moule étanche au niveau des orifices de sortie (4), qui, pendant la mise en oeuvre, peut être totalement ou partiellement enlevé de la zone de mélange (5), et qui libère totalement ou partiellement les orifices de sortie (4).

13. Procédé pour la fabrication in situ de formulations composées d'au moins deux composants où les composants présents dans les contenants d'approvisionnement séparés sont mélangés immédiatement avant l'emploi de la formulation à l'aide d'un système d'emballage selon l'une des revendications 1 à 13.

14. Procédé selon la revendication 13, **caractérisé en ce que** la vitesse d'introduction du flux de réactif dans la zone de mélange (5) est plus élevée que la vitesse d'écoulement du produit dans la zone de mélange (5).

15. Procédé selon l'une des revendications 13 à 14, **caractérisé en ce que** la formulation est une micro ou une nano émulsion.

16. Procédé selon l'une des revendications 13 à 15, **caractérisé en ce que** l'un des composants est une phase aqueuse, liquide et que l'autre composant est une phase hydrophobe, liquide ou une phase contenant une substance sensible à l'eau ; ou que les composants contiennent des substances qui réagissent chimiquement lorsqu'elles entrent en contact ou qui modifient la consistance physique du mélange.

17. Procédé selon l'une des revendications 13 à 16 pour la fabrication de colorants, de produits de collage, de produits alimentaires, de produits pharmaceutiques, de produits cosmétiques ou de produits de construction ou d'entretien.

18. Procédé selon la revendication 17 pour la fabrication de préparations sous forme d'émulsions contenant des préparations contenant au moins un agent actif cosmétique pour les cheveux ou la peau, dermatologique ou pharmaceutique, des produits fixateurs pour les cheveux, des produits colorants pour les cheveux ou des produits pour des permanentes.
